Europäisches Patentamt

European Patent Office

Office européen des brevets

(19)

(11) Publication number: **0 132 021 B1**

# EUROPEAN PATENT SPECIFICATION

(45) Date of publication of patent specification: **30.10.91**

(51) Int. Cl.⁵: **C07K 7/00, A61K 37/02, G01N 33/53**

(21) Application number: **84302819.2**

(22) Date of filing: **26.04.84**

The file contains technical information submitted
after the application was filed and not included in
this specification

(54) **TGF polypeptides, antigenic oligopeptides derived therefrom and antibodies produced therefrom.**

(30) Priority: **09.05.83 US 492751**
**12.04.84 US 598136**

(43) Date of publication of application:
**23.01.85 Bulletin 85/04**

(45) Publication of the grant of the patent:
**30.10.91 Bulletin 91/44**

(84) Designated Contracting States:
**AT BE CH DE FR GB IT LI LU NL SE**

(56) References cited:
**WO-A-84/01106**
**US-A- 4 287 185**

**PROCEEDINGS OF THE NATIONAL ACADEMY OF SCIENCES (USA), vol. 80, August 1983; H. MARQUARDT et al. "Transforming growth factors produced by retrovirus-transformed rodent fibroblasts and human melanoma cells: Amino acid sequence homology with epidermal growth factor", pages 4684-4688 * page 4686; figure 2 ***

**Chemical Abstracts vol. 95, no. 21, 23 November 1981, Columbus, Ohio, USA; A.B.**

**ROBERTS et al. "New class of transforming growth factors potentiated by epidermal growth factor: Isolation from non-neoplastic tissues", page 129, column 2, abstract no. 181524d & Proc. Natl. Sci. USA, vol. 78, no. 9, 1981 pages 5339-5343**

(73) Proprietor: **Todaro, George Joseph**
**1940 15th Avenue East**
**Seattle Washington 98112(US)**

(72) Inventor: **Todaro, George Joseph**
**1940 15th Avenue East**
**Seattle Washington 98112(US)**

(74) Representative: **Ruffles, Graham Keith et al**
**MARKS & CLERK 57-60 Lincoln's Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to biologically active polypeptides and their production from natural or synthetic sources, oligopeptides derived from said polypeptides, and compositions and methods for usefully applying the biological activity associated with the polypeptides and oligopeptides derived therefrom in the health sciences field. More particularly, this invention is directed to a general polypeptide structure which defines proteins having cell growth promoting activity and a novel class of transforming growth factor (TGF) polypeptides which have the property of reversibly conferring the transformed phenotype on normal cells in vitro and thus appear to be proximate effectors of the malignant phenotype. In another particular aspect, this invention is directed to antigenic oligopeptides derived from the TGF polypeptides and to antibodies raised to the antigenic oligopeptides and the associated TGF polypeptides which are useful in the detection and treatment of malignancies. In a further particular aspect, this invention is directed to a class of oligopeptides which have the ability to bind to cellular growth factor receptors and thus to potentially interfere with transformation of certain cell lines into a cancerous state. In a still further particular aspect, this invention is directed to a process for isolating TGF polypeptides in homogeneous form from both transformed human and murine cell lines and body fluids and to the homogeneous TGF polypeptides so obtained. Other particular aspects of this invention are directed to compositions and methods for detection and treatment of cancer and other proliferative diseases and for cell growth associated treatment, for example, wound healing and ulcer treatment.

A number of polypeptide hormone and hormone-like growth factors have been found in tissue fluids and their relationship in the control of normal cellular growth or mitosis has been established. These mitogenic polypeptide growth factors include insulin, insulin-like growth factors, platelet-derived growth factor, nerve growth factor, fibroblast growth factor and epidermal growth factor (EGF). At least some of these known growth factors have an effect on the growth of transformed cells, however, on the basis of in vitro tests, it appears that transformed cells require less of these known growth factors for optimal growth and multiplication than do normal cells. In particular, it has been shown in experiments in cell culture, that the addition of exogenous growth factors such as insulin and EGF can cause normal cells to mimic certain changes in cellular properties that are analogous to transformation; however, they are unable to produce all of the changes associated with the transformed phenotype, e.g., see Sporn et al., (1981) The New Eng. J. of Med., No. 15, pp. 878-880.

Recently, new types of polypeptide growth factors designated as transforming growth factors or TGFs have been found in certain human and animal carcinoma and sarcoma cells which possess a greater complement of the properties apparently essential to phenotypic transformation (Roberts et al. (1980) Proc. Natl. Acad. Sci. USA 77, pp. 3494-3498 and Todaro et al. (1980) Proc. Natl. Acad. Sci. USA 77, pp. 5258-5262). The isolation of partially purified TGFs from non-neoplastic tissues of the mouse, more particularly the submaxillary glands of male mice, has been reported (Roberts et al (1981) Proc. Natl. Acad. Sci, USA 78 pp 5339-5343). The TGF polypeptides as a class are characterized by the changes which they cause when applied to untransformed, non-neoplastic indicator cells growing in culture. These changes include a) loss of density-dependent inhibition of cell growth in monolayer culture, b) overgrowth of cells in monolayer culture, c) change in cellular shape, with the result that the indicator cells assume the neoplastic phenotype, and d) acquisition of anchorage-independence, with the resultant ability to grow in soft agar. The property of anchorage-independent growth of cells in culture has a particularly high correlation with neoplastic growth in vivo. At least certain of the TGF polypeptides show some relationship with EGF in that they are both heat-stable, acid-stable peptides sensitive to reducing agents and proteases and they appear to specifically interact with, and produce biological effects through, cellular membrane EGF receptors, TGF competing with EGF for binding to the cellular EGF receptor. However, TGF is distinguishable from EGF in several important respects. In particular, EGF does not induce anchorage-independent growth of cells in culture nor do antibodies to EGF detect TGF in either radioimmunoassay or immunoprecipitation tests. Further, EGF has only a slight effect on the phenotype of cultured cells, whereas TGF produces a more pronounced phenotypic alteration in cultured cells and confers on them the ability to behave as transformed cells. Interestingly, the transformation produced by TGF is not permanent but reversible in the absence of TGF and there is no evidence that TGF acts as a complete carcinogen itself. (Todaro et al. (1981) J. of Supramolecular Structure and Cell Biochem. 15, pp. 287-301).

Thus, TGF polypeptides are a unique class of proteins distingishable from other growth factors such as EGF from the standpoint of both biological properties and chemical structure. These TGFs, in turn, possess a variety of properties of value or potential value in the health sciences field including potent but reversible cell growth or mitogenic properties which find use in cell repair including, for example, wound healing and ulcer therapy. Additionally, the production of TGF polypeptides, or elevated levels of production, are characteristic of, if not essential to, the morphologic transformation of certain cell lines in both human and murine tissue and/or fluids; therefore, the TGF polypeptides or antigenic fragments thereof are of value in differentiating normal cells from tumor cells and antibodies raised thereto have application in both the diagnosis and treatment of malignancies. Further, realization that certain TGF polypeptides specifically interact with and produce their biological effects through cellular membrane EGF receptors raises the possibility, once the basic TGF polypeptide structure is determined, of correlating the structure with the structure of EGF to develop oligopeptides having chemical characteristics to allow binding to the EGF receptors without concomitant phenotypic transformation of the cell. Oligopeptides having this characteristic EGF receptor binding ability find application in treatment of malignancies since the oligopeptide will interfere or compete with TGF for available receptor sites and thereby interrupt the expression of the transformed properties of the cell.

With the present invention, a method has been developed to obtain TGF polypeptides in sufficient quantity and purity to allow complete structure determination and as a result of this determination and other observations, including the finding of substantial homology between human and murine TGFs, a basic peptide structure has been discovered which has broad application in cell mitosis and the cell growth related field of use. Further, homogeneous TGF polypeptides are obtained having application in both the cell growth field and in the detection and treatment of cancer and other proliferative diseases. Additionally, antigenic oligopeptides and oligopeptides having the ability to bind to cellular growth factor receptors are derived from the basic peptide structure and the determined TGF polypeptide structures. Finally, compositions and methods, including antibodies raised to the TGF polypeptides and antigenic oligopeptides derived therefrom are provided for use in the health sciences field.

Marquardt and Todaro, J. of Bio. Chem. (1982) Vol. 257, No. 9, pp. 5220-5225, (published May 10, 1982) describe the isolation of a low molecular weight human TGF from serum-free medium conditioned by a human metastatic melanoma tumor line by a sequence of process steps including extraction in 1M acetic acid and sequential purification on reversed phase high pressure liquid chromatography eluting first with acetonitrile solvent followed by elution with 1-propanol to afford a purified TGF having characteristic TGF biological activity, e.g., induction of anchorage-independent cell growth. Twardzik et al. Science (1982) Vol. 216, pp. 894-897 (published May 21, 1982) report the use of the same purification methodology to purify a TGF from a virus transformed rat cell. The biological activity of the purified material in the cell culture is also demonstrated. Pike et al. J. of Bio. Chem. (1982) Vol. 257, No. 24, pp. 14628-14631 (published December 25, 1982) disclose that both partially purified rat and human TGF have the ability to activate a protein kinase in human tumor cell membranes and therefore to stimulate phosphorylation of a synthetic tyrosine-containing peptide. The only other molecules so far described that have this activity are EGF, insulin and platelet derived growth factor, all of which are believed to have important physiologic functions in man and animal. Other references of interest are cited in the aforementioned articles.

A basic protein structure has now been found which defines a new class of biologically active molecules. The finding of this framework polypeptide affording biological activity, particularly in the cell growth promotion area, is based on the discovery that a definite correlation exists between the three dimensional structure of certain polypeptides, including TGFs, containing multiple disulfide bonds and the biological activity attributable to the polypeptide. Accordingly, in one of its broadest aspects, the present invention is directed to biologically active polypeptides containing at least one peptide sequence of the formula I:

-Cys-(AA)$_a$-Cys-(AA)$_b$-Cys-(AA)$_c$-Cys-AA-Cys-(AA)$_d$-Cys-    I

wherein AA is an amino acid residue selected from Val, Ser, His, Phe, Asn, Lys, Asp, Thr, Gln, Arg, Leu, Glu, Pro, Ala, Gly, Trp and Tyr, and a is 7, b is 4 or 5, c is 10, and d is 8. Also contemplated are compounds according to formula I wherein when b is 4, AA may also be Ile or Met, in addition to the amino acid residues recited.

Another aspect of the present invention is directed to a specific class of polypeptides having transforming growth factor properties which include compounds of the formula II or oligomers thereof:

```
                    5                      10
Val-Val-Ser-His-Phe-Asn-R-Cys-Pro-Asp-Ser-His-Thr-
        15                    20                    25
Gln-R'-Cys-Phe-His-Gly-Thr-Cys-Arg-R''-Leu-Val-Gln-
              30                      35              II
Glu-Glu-Lys-Pro-Ala-Cys-Val-Cys-His-Ser-Gly-R'''-
        40                    45                    50
Val-Gly-Val-Arg-Cys-Glu-His-Ala-Asp-Leu-Leu-Ala
```

wherein R is Asp or Lys, R' is Phe or Tyr, R" is Ser or Phe, and R'" is Phe or Tyr. Also within the scope of the invention are antigenic oligopeptides derived from the polypeptides of formula II.

An additional aspect of this invention relates to polypeptide growth factors containing one or more of the following peptide fragments:

A.   Val-Val-Ser-His-Phe-Asn-Lys-Cys-Pro-Asp-Ser-
     His-Thr-Gln-Tyr-Cys-Phe-His-Gly-Thr-Cys

B.   Val-Val-Ser-His-Phe-Asn-Asp-Cys-Pro-Asp-Ser-
     His-Thr-Gln-Phe-Cys-Phe-His-Gly-Thr-Cys

C.   Leu-Val-Gln-Glu-Glu-Lys-Pro-Ala-Cys-Val-Cys-
     His-Ser-Gly, and

D.   Cys-His-Ser-Gly-Tyr-Val-Gly-Val-Arg-Cys-Glu-
     His-Ala-Asp-Leu-Leu-Ala.

A still further aspect of the present invention is directed to a class of oligopeptides which have an ability to bind cellular growth factor receptors, and therefore, have potential utility in treatment of malignancy. This class of oligopeptides is based on the discovery of key sequences in larger polypeptides molecules which exhibit both significant amino acid sequence homology in the appropriate three dimensional structure and have the ability to bind to cellular growth receptor sites. Accordingly, this aspect of the invention provides oligopeptides having the formula III:

$(AA)_x$-Cys-$(AA)_y$-Gly-R-$(AA)_z$-Arg-Cys-$(AA)_{z'}$,    III

wherein R is Phe or Tyr and AA is an amino acid residue selected from Val, Asn, His, Ser, Lys, Ile, Gly, Leu, Asp, Asn, Cys, Thr, Ala, Tyr, Pro, Glu, Gln, and Arg, and x is 0 or an integer of from 1 to 5, y is 2, z is 3, and z' is 0 or an integer of from 1 to 6.

Also contemplated by the invention are biologically active compositions and methods using the polypeptide and oligopeptide structures given above including antibodies to the polypeptides of formula II and the antigenic oligopeptides derived therefrom, said antibodies being optionally labeled with a label capable of providing a detectable signal for use in diagnostic methods or labeled with a cytotoxic agent for use in cancer or proliferative disease therapy. Other compositions and methods utilizing the cell growth promoting properties of the polypeptides of the present invention also form part of the present invention.

A final aspect of the present invention includes a process for isolating homogeneous transforming growth factor polypeptides from less pure aqueous solutions containing said polypeptides, including body fluids and aqueous mediums conditioned with transforming growth factor-producing cell lines, as well as the homogeneous transforming growth factor polypeptides produced thereby and antibodies raised to said homogeneous polypeptides. In its broadest aspects, the process of the invention involves isolation of a homogeneous transforming growth factor polypeptide from an aqueous medium containing said transforming growth factor polypeptide in impure form by the process steps comprising:

(1) dialyzing the aqueous medium containing the transforming growth factor in impure form against aqueous acetic acid to afford a solvent phase containing transforming growth factor polypeptide which phase is concentrated and optionally clarified,

(2) reconstituting the concentrated solvent phase of step 1) with aqueous acetic acid and subjecting the reconstituted solution to gel permeation chromatography by applying reconstituted solution to a gel permeation chromatography column conditioned with aqueous acetic acid and eluting with aqueous acetic acid to obtain selected fractions of eluate containing transforming growth factor polypeptide in an enhanced state of purity, said selected fractions being combined and concentrated, to afford a partially purified, transforming growth factor polypeptide-containing product.,

(3) subjecting the partially purified, transforming growth factor polypeptide-containing product of step 2) to sequential reverse phase high pressure chromatography by passing said product, after reconstitution in aqueous trifluoroacetic acid, through one or more hydrocarbon bonded silica matrix colums, which have been equilibrated with aqueous trifluoroacetic acid, under high pressure liquid chromatography conditions, the initial column elution being performed using a linear acetonitrile gradient in aqueous trifluoroacetic acid and the subsequent column elution, which is carried out on the combined, transforming growth factor polypeptide-containing fractions on the initial high pressure chromatography step, being performed using a linear 1-propanol gradient in aqueous trifluoroacetic acid, said 1-propanol gradient being increased in sufficiently small 1-propanol concentration increments to afford the transforming growth factor polypeptide as a single distinct peak in the state of a homogeneous polypeptide.

The peptide sequence which characterizes the basic protein structure according to the invention contains six cysteine residues positioned at critical positions in the polypeptide framework. It is speculated that the positioning of the cysteine residues allows the polypeptide to fold in a particular fashion as a result of disulfide bridges between paired cysteines, and therefore to present a three dimensional structure which contributes to the biological activity of the resulting protein. There is some evidence suggesting a particular disulfide bridging sequence wherein (numbering the Cys residues in formula I above 1 through 6 going from left to right) Cys-1 is bonded to Cys-3, Cys-2 is bonded to Cys-4 and Cys-5 is bonded to Cys-6 by disulfide bonds in biologically active forms of the basic protein structure. The exact chemical nature of the amino acid residues recited for the amino acid sequences spaced between the Cys residues in formula I do not appear to be particularly critical provided at least 10 different amino acids from the group recited for formula I are employed and no amino acid is repeated more than three times as consecutive residues in any given sequence. Of the amino acid residues listed for formula I, preference is given to Val, Ser, His, Phe, Lys, Asp, Thr, Gln, Leu, Glu, Pro, Ala, and Gly. A preferred group of biologically active polypeptides having at least one peptide sequence of formula I are polypeptides and oligomers thereof of the formula IV:

$$(AA)_n\text{-Cys-}(AA)_m\text{-Cys-}(AA)_o\text{-Cys-}(AA)_p\text{-Cys-AA-Cys-}(AA)_q\text{-Cys-}(AA)_r \qquad\qquad IV$$

wherein AA is an amino acid residue selected from Val, Ser, His, Phe, Asn, Lys, Asp, Thr, Gln, Arg, Leu, Glu, Pro, Ala, Gly, Trp and Tyr, and n is an integer of from 4 to 10, m is 7, o is 4 or 5, p is 10, q is 8 and r is an integer of from 6 to 12. In this preferred group, even further preference is given to polypeptides or oligomers thereof wherein o is 4 and n and r are 7. With this preferred group, the amino acid residues designated by AA may also be Ile and/or Met, in addition to the amino acid residues recited previously for formula IV. Most preferred are polypeptides wherein the amino acid residues in the sequences spaced between the Cys residues are selected from Val, Ser, His, Phe, Lys, Asp, Thr, Gln, Leu, Glu, Pro, Ala and Gly. Typically the polypeptides in accordance with formulas I and IV will have molecular weights ranging from about 5,000 to about 35,000. Preferred polypeptides in this respect have molecular weights in the range of 5,000 to 8,000.

As noted above, the present invention also contemplates a new class of TGF polypeptides and oligomers thereof of the formula (formula II above):

$$\begin{array}{c} \overset{5}{} \qquad\qquad\qquad \overset{10}{} \\ \text{Val-Val-Ser-His-Phe-Asn-R-Cys-Pro-Asp-Ser-His-Thr-} \\ \overset{15}{} \qquad\qquad\qquad \overset{20}{} \qquad\qquad\qquad \overset{25}{} \\ \text{Gln-R'-Cys-Phe-His-Gly-Thr-Cys-Arg-R''-Leu-Val-Gln-} \\ \overset{30}{} \qquad\qquad\qquad \overset{35}{} \qquad\qquad\qquad \text{II} \\ \text{Glu-Glu-Lys-Pro-Ala-Cys-Val-Cys-His-Ser-Gly-R'''-} \\ \overset{40}{} \qquad\qquad\qquad \overset{45}{} \qquad\qquad\qquad \overset{50}{} \\ \text{Val-Gly-Val-Arg-Cys-Glu-His-Ala-Asp-Leu-Leu-Ala} \end{array}$$

wherein R is Asp or Lys, R' is Phe or Tyr, R" is Ser or Phe, and R'" is Phe or Tyr. This novel class of polypeptides is derived from the finding that certain TGFs obtained from a variety of mammalian species (both murine and human) have substantial homology in the amino acid make-up of thepeptide sequence (greater than 90% of the sequences being identical) as well as substantially the same biological properties. In particular, TGF polypeptides in accordance with the formula given above cause the loss of density-dependent inhibition of cell growth in monolayer culture, overgrowth in monolayer culture, characteristic change in cellular morphology and acquisition of anchorage-independent growth when applied to untransformed, non-neoplastic indicator cells grown in culture. In addition to being extremely potent cell growth promoters and effectors of cell transformation, the TGF polypeptides in accordance with the above formula compete with EGF for binding to the cellular EGF receptor and also have the ability to activate an enzyme, a protein kinase, in human tumor cell membranes. Preferred TGF polypeptides of formula II above include those wherein R is Asp, R' is Phe, R" is Ser, and R'" is Phe, or where R is Lys, R' is Tyr, R" is Phe, and R'" is Tyr. As will be discussed in greater detail below, these TGF polypeptides may be suitably obtained from a variety of transformed human and murine cell lines, certain embryonic cell lines and body fluids of tumor-carrying mammals using the isolation process of the invention or by conventional synthetic or recombinant means for synthesizing polypeptides. Typically the molecular weight of the TGF polypeptides and oligomers thereof of formula II will be in the range of from about 5,000 to about 35,000. In this regard, preference is given to TGF polypeptides having a molecular weight of about 5,000 to 8,000.

Recognition of the substantial peptide sequence homology in the novel class of TGF polypeptides of formula II above and the commonality of biological properties associated therewith allows for further definition of a class of polypeptide growth factors which are within the scope of the present invention. These polypeptide growth factors are defined as containing one or more of the following peptide fragments:

    **A.**    **Val-Val-Ser-His-Phe-Asn-Lys-Cys-Pro-Asp-Ser-His-Thr-Gln-Tyr-Cys-Phe-His-Gly-Thr-Cys**

    **B.**    **Val-Val-Ser-His-Phe-Asn-Asp-Cys-Pro-Asp-Ser-His-Thr-Gln-Phe-Cys-Phe-His-Gly-Thr-Cys**

    **C.**    **Leu-Val-Gln-Glu-Glu-Lys-Pro-Ala-Cys-Val-Cys-His-Ser-Gly, and**

    **D.**    **Cys-His-Ser-Gly-Tyr-Val-Gly-Val-Arg-Cys-Glu-His-Ala-Asp-Leu-Leu-Ala.**

Preferred polypeptides in this respect, include polypeptides containing a combination of peptide fragments A and C and polypeptides containing a combination of peptide fragments B and C. Most preferred are polypeptides containing fragments B, C and D. Here again, the polypeptide growth factors containing one or more of peptide fragments A, B, C and D will generally have a molecular weight in the range of from about 1,000 to about 35,000 preferably from 1,000 to 8,000. The lower end of the molecular weight range would include the above specified peptide fragments themselves as complete polypeptides having the characteristic growth factor biological activity.

Previously it has been noted that the TGF polypeptides of the present invention are of value in the detection of malignancies in mammals since the production and/or elevated levels of production of the TGF polypeptides are characteristic of morphologic transformation of certain human and murine cell lines. In this regard, antibodies to the TGF polypeptides have utility in diagnosis of malignancy since they can be used to detect extremely low levels of TGF polypeptide present in tumor cells or in body fluids. While the entire TGF polypeptide molecule can be used to generate antibodies (both polyclonal and monoclonal), it is also possible, and advantageous from the standpoints of cost and technical effort, to determine various regions in the TGF polypeptide sequence which are likely to be determinant sites and to use these oligopeptides of at least about eight amino acids, typically at least about 10 and not more than about 20 amino acids, to define a hapten which can be used to induce antibody formation. As further discussed below, the oligopeptide is bound to an appropriate immunogen and introduced into a vertebrate to produce the desired antibodies. Accordingly, the present invention also provides a series of oligopeptides corresponding to antigenic regions in the TGF polypeptides. Exemplary species of the antigenic oligopeptides useful in generating antibodies in accordance with the present invention are listed below.

A.    Val-Val-Ser-His-Phe-Asn-Asp-Cys-Pro-Asp-His-Thr

B.    Val-Val-Ser-His-Phe-Asn-Lys-Cys-Pro-Asp-His-Thr

C.    Arg-Phe-Leu-Val-Gln-Glu-Glu-Lys-Pro-Ala

D.    Arg-Tyr-Leu-Val-Gln-Glu-Glu-Lys-Pro-Ala

E.    Cys-His-Ser-Gly-Phe-Val-Gly-Val-Arg-Cys-Glu-His-Ala-Asp-Leu-Leu-Ala

F.    Cys-His-Ser-Gly-Tyr-Val-Gly-Val-Arg-Cys-Glu-His-Ala-Asp-Leu-Leu-Ala

Another compositional aspect of the present invention is directed to a class of oligopeptides which have therapeutic value in treatment of malignancies. These oligopeptides have the ability to bind to cellular growth factor receptors without causing phenotypic transformation of the cell and therefore they can effectively compete with TGF polypeptides for available receptor sites on the cell and interrupt or minimize cell transformation which is characteristic of TGF binding to cell receptors. The oligopeptides according to the invention which have the ability to bind to cellular receptors are of the formula (formula III above):

$(AA)_x$-Cys-$(AA)_y$-Gly-R-$(AA)_z$-Arg-Cys-$(AA)_{z'}$,

wherein R is Phe or Tyr and AA is an amino acid residue selected from Val, Asn, His, Ser, Lys, Ile, Gly, Leu, Asp, Asn, Cys, Thr, Ala, Tyr, Pro, Glu, Gln, and Arg, and x is 0 or an integer of from 1 to 6, y is 2, z is 3 and z' is 0 or an integer of from 1 to 6. Preferred oligopeptides in accordance with the above formula include those wherein x and z' are 0 and AA is an amino acid residue selected from Val, His, Ser, Ile, Gly and Asp. A desirable group of biologically active oligopeptides related to those of formula II are those containing two glycine residues in addition to afford a sequence of the following formula:

$(AA)_x$-Cys-$(AA)_2$-Gly-$(AA)_2$-Gly-$(AA)_2$-Cys-$(AA)_z$,

wherein the amino acid residues designated by the AA's are the same as those mentioned for formula III above but including Phe and subscripts x and z' are as given for formula III above. These oligopeptides assume a common three dimensional structure attributable to the disulfide bridging between the two cysteines. This disulfide bridge characterizes the biologically active forms of the oligopeptides. Particularly preferred oligopeptides in this regard are selected from the class consisting of

1. Cys-His-Ser-Gly-Tyr-Val-Gly-Val-Arg-Cys and
2. Cys-His-Ser-Gly-Phe-Val-Gly-Val-Arg-Cys

The polypeptides and oligopeptides according to the invention as defined by the structural formulas (formulas I through IV) and peptide sequences given above can be prepared by synthetic techniques, techniques whereby the peptide is isolated from a naturally occurring source, e.g., cell lines and body fluids, and by techniques employing hybrid DNA technology. For those polypeptides and oligopeptides of the invention containing up to about 50 amino acid residues, conventional solid phase peptide synthesis is suitably employed. In this general synthetic procedure for making peptides, which is described, for example, in U.S. Patent 4,341,761 to Ganfield et al., employs known side-chain protecting groups and conventional polystyrene resins supports - e.g., chloromethylated resins, hydroxymethyl resins or benzhydrylamine resins - to affect the amino acid coupling. For polypeptides containing in excess of about 50 amino acid residues, the process according to the invention for isolating homogeneous TGFs from natural sources which is described in detail below can be suitably employed to obtain pure forms of the desired peptide. In this regard, particularly suitable sources of the TGF polypeptides according to the invention include serum-free medium conditioned by retrovirus-transformed Fischer rat embryo fibroblasts, in particular fibroblasts transformed with Snyder-Theilen feline sarcoma virus, Moloney murine sarcoma virus-transformed mouse 3T3 cells and human metastatic melanoma cell lines A2058 and A375. Sources and methods for suitable murine cell lines are described in DeLarco et al., (1980) J. Biol. Chem. 255, pp. 3685-3690 and Ozanne et al., (1980) J. Cell. Physiol 105, pp. 163-180. Sources and methods for human cell lines are similarly described in Todaro et al., (1980) Proc. Natl. Acad. Sci. USA 77, pp. 5258-5262 and Giard et al. (1973) J. Natl. Cancer Inst., 51, pp. 1417-1423. The isolation process of the invention described below can also be used to obtain TGF polypeptides according to the invention from various body fluids such as urine, serum, plasma, whole blood or cerebrospinal fluid of human or murine subjects carrying malignancies, or transformed cells which produce TGF polypeptides. In this regard, a suitable source of TGF polypeptides according to the invention is the urine or other body fluids of mice which have been inoculated with tumor cells (human melanoma or transformed rat) known to produce TGF polypeptides. In all cases the identification and purity of the TGF polypeptide can be monitored by a radioreceptor assay based on receptor cross-reactivity with EGF (see experimental examples below). In techniques utilizing recombinant or hybrid DNA technology, the oligopeptides according to the invention or segments of the polypeptides according to the invention containing up to, for example, 20 amino acids can be used to deduce the codon sequence for single stranded nucleotide (DNA) probes. These nucleotide probes can then be synthesized using known synthetic techniques and used as a probe to obtain messenger RNA coding for growth factor-type polypeptides in both normal and transformed cells or body fluids containing said peptides. Once messenger RNA is obtained, conventional techniques can be used for reverse transcribing of the mRNA to cDNA and subsequent cloning of the cDNA in a suitable vector to obtain expression of the desired polypeptide.

The process according to the invention provides a uniquely effective means of obtaining TGF polypeptides in homogeneous form from various aqueous based fluids containing less pure forms of the TGF polypeptides such as serum-free mediums conditioned by transformed cell lines which produce TGF polypeptides or body fluids, e.g., urine from mammals carrying malignancies or transformed cells which produce the TGF polypeptides. Important aspects of this unique isolation or purification process include an initial extraction or dialysis step using aqueous acetic acid, subsequent gel permeation chromatography of the acid-soluble TGF-containing activity, and finally, reverse phase high pressure liquid chromatography using sequentially acetonitrile and 1-propanol in the presence of aqueous trifluoroacetic acid. Broadly defined, this process involves isolation of a homogeneous transforming growth factor polypeptide from an aqueous medium containing said transforming growth factor polypeptide in impure form by the process steps comprising:

(1) dialyzing the aqueous medium containing the transforming growth factor in impure form against aqueous acetic acid to afford a solvent phase containing transforming growth factor polypeptide which phase is concentrated and optionally clarified,

(2) reconstituting the concentrated solvent phase of step 1) with aqueous acetic acid and subjecting the reconstituted solution to gel permeation chromatography by applying reconstituted solution to a gel permeation chromatography column conditioned with aqueous acetic acid and eluting with aqueous acetic acid to obtain selected fractions of eluate containing transforming growth factor polypeptide in an enhanced state of purity, said selected fractions being combined and concentrated, to afford a partially purified, transforming growth factor polypeptide-containing product,

(3) subjecting the partially purified, transforming growth factor polypeptide-containing product of step 2)

8

to sequential reverse phase high pressure chromatography by passing said product, after reconstitution in aqueous trifluoroacetic acid, through one or more hydrocarbon bonded silica matrix columns, which have been equilibrated with aqueous trifluoroacetic acid, under high pressure liquid chromatography conditions, the initial column elution being performed using a linear acetonitrile gradient in aqueous trifluoroacetic acid and the subsequent column elution, which is carried out on the combined, transforming growth factor polypeptide-containing fractions of the initial high pressure chromatography step, being performed using a linear 1-propanol gradient in aqueous trifluoroacetic acid, said 1-propanol gradient being increased in sufficiently small 1-propanol concentration increments to afford the transforming growth factor polypeptide as a single distinct peak in the state of a homogeneous polypeptide.

In a preferred application, the process according to the invention is employed to isolate homogeneous TGFs from serum-free media conditioned by transformed, TGF-producing cell lines. In this preferred application, the conditioned medium is suitably clarified, e.g., by centrifugation, and concentrated prior to dialysis and the TGF-containing solvent phase from dialysis is suitably clarified, e.g., by centrifugation, as well as concentrated prior to gel permeation chromatography. In any case, the dialysis is suitably carried out using an aqueous acetic acid solvent having an acetic acid concentration of from 0.01 to 1 molar, with 0.1 molar acetic acid being preferred. The gel permeation chromatography may be carried out using a variety of gels conventionally employed to separate proteins or polypeptides based on molecular size. Suitable gels include dextran gels, agarose gels and polyacrylamide gels. In this regard, preference is given to polyacrylamide gel filtration resins (Bio-Gel®) such as Bio-Gel P-10, Bio-Gel P-30 and Bio-Gel P-60, Bio-Gel P-10 being especially preferred. The aqueous acetic acid used to condition the column and to elute the TGF-containing fractions suitably has an acetic acid concentration of from 0.2 to 2.0 molar, with 1.0 molar acetic acid being preferred. The TGF-containing fractions which elute from the column can be identified by determining their EGF-competing activity and growth promotion activity in soft agar (see experimental examples below). After gel permeation chromatography, the fractions containing TGF polypeptides in an enhanced state of purity are pooled together and concentrated, for example, by lyophilization as a preparative step for further purification by reverse phase high pressure liquid chromatography (HPLC).

The final stage of the purifiction process of the invention involves sequential HPLC with acetonitrile and 1-propanol in the presence of aqueous trifluoroacetic acid. This sequential HPLC can be carried out using one or more HPLC columns but it is preferred to carry out the sequential HPLC steps using a single HPLC column. The column packing employed is suitably a porous silica matrix to which a long chain hydrocarbon, for example, hydrocarbon containing 16 to 22 carbon atoms, is bound. Preferred packings are μBondapak hydrocarboncolumns, in particular μBondapak $C_{18}$ column (10-μm particle size, 0.39 x 30 cm, Waters Associates). Typically, the procedure is carried out under pressure, preferably in the range of from about 50 to about 5,000 psi. Prior to application to the column, the concentrated TGF-containing fractions are reconstituted in an aqueous 1 to 10% trifluoroacetic acid and adjusted to a pH in the range of 2 to 5, preferably 3.5 by the addition of trifluoroacetic acid. The column is suitably equilibrated with 0.01 to 0.1% aqueous trifluoroacetic acid, preferably 0.05% aqueous trifluoroacetic acid, before sample injection. The first elution is carried out with acetonitrile in a 0.01 to 0.1%, preferably 0.05% trifluoroacetic acid using a linear acetonitrile gradient (acetonitrile concentration increased linearly at a gradient in the range of about 0.1%/min to about 1%/min). The elution is carried out over a time period of from 0.2 to 3 hours at a flow rate of about 0.2 to 2 ml/min and at a temperature of from 10 to 50° C, preferably about 40° C. The pooled fractions containing TGF activity as determined by EGF competition and soft agar assay are concentrated, for example, by lyophilization, prior to the second step of the HPLC using 1-propanol solvent. For the second step of the sequential HPLC, the pooled and concentrated fractions from the first HPLC elution are reconstituted in 0.01 to 0.1% trifluoroacetic acid and rechromatographed on the same column or a second column equilibrated with trifluoroacetic acid ina manner identical to that used for the first column. This second elution is carried out with 1-propanol ina 0.01 to 0.1%, preferably 0.035% trifluoroacetic acid using a linear 1-propanol gradient. It is important for optimum results to employ a shallow linear 1-propanol gradient in this step. In particular, the 1-propanol concentration should be increased linearly at a gradient which does not exceed 0.1%/min and preferably the linear 1-propanol gradient should be maintained between 0.01%/min and 0.05%/min during the elution. This second elution is suitably carried out over a time period of from 1 to 5 hours at a flow rate of about 0.5 to 5 ml/min and at a temperature of from 10 to 60° C, preferably about 40° C. By controlling the linear 1-propanol concentration gradient at the shallow levels given above, it is possible to elute TGF polypeptides as well-defined peaks of TGF activity in the form of homogeneous polypeptides.

With the isolation process of the invention, it is possible to recover up to about 70% of the initial TGF activity in the impure starting matrial while achieving degrees of purification in excess of 200,000-fold. The homogeneous TGF polypeptides obtained by the isolation process of the invention typically have molecular weights in the range of about 5,000 to about 35,000 and are of sufficient purity to permit peptide sequencing. Preferred homogeneous TGF polypeptides which are obtained with theprocess of the invention include TGFs having apparent molecular weights of 7,400, 20,000 and 30,000 to 35,000. These homogeneous TGF polypeptides show characteristic biological properties of TGF polypeptides when they are applied to untransformed, non-neoplastic indicator cells growing in culture including acquisition of anchorage-independence, with the resultant ability to grow in soft agar. Further the homogeneous TGF polypeptides can be used to raise antibodies (see below) which have value in the detection and treatment of cancer and other proliferative diseases in accordance with the invention. In this regard, it is not essential that each of the forms of the TGF be purified to homogeneity in order to produce antibodies to the different TGF peptides. These various forms of antibodies to the TGF polypeptides are also contemplated by this invention.

Antibodies according to the invention include both monoclonal and polyclonal antibodies raised to the TGF polypeptides of formula II given above, antigenic oligopeptides derived from the TGF polypeptides of formula II and the homogeneous TGF polypeptides obtained using the isolation process of the invention from various transformed cell lines and body fluids of mammals carrying malignancies or transformed cells. The antibodies according to the invention can be prepared in a variety of ways known in the art, depending on whether monoclonal or polyclonal antibodies are desired. For polyclonal antibodies, a vertebrate, typically a domestic animal, is hyperimmunized with antigen and the blood collected shortly after repeat immunizations and the gamma globulin isolated. Suitable methods for preparing polyclonal antibodies are described in the Handbook of Experimental Immunology, 3rd edition, Weir, Editor, Blackwell Scientific publications, Oxford and London, 1978. For monoclonal antibodies, a small animal, typically a mouse or rat, is hyperimmunized with antigen, the spleen removed and the lymphocytes fused with myeloma cells in the presence of a suitable fusion promoter. The resulting hybrid cells or hybridomas are screened to isolate individual clones, each of which secrete a single antibody species to the antigen. The individual antibody species obtained in this way are each the product of a single B cell from the immune animal generated in response to a specific antigenic site recognized on the immunogenic substance. The general process for obtaining monoclonal antibodies, including those according to the invention, is described by Kohler and Milstein (1975) Nature 256, pp. 495-497. The polypeptides and antigenic oligopeptides of the invention employed directly in the immunization procedure or they may be bound to a suitable carrier-protein using methods known in the art, for example, see U.S. Patent 4,341,761 to Ganfield et al. Use of a carrier protein is particularly preferred when the immunization is carried out using the antigenic oligopeptides of the invention.

The antibodies according to the invention may be used in a variety of ways. In a preferred application, they may be used for diagnosis of malignancy and other proliferative diseases. In instances where the antigen may be found in a physiological fluid or at a concentration differential only when malignancy or other proliferative disease exists, the physiological fluid, such as serum plasma, whole blood or cerebrospinl fluid may be assayed. Antibodies employed in assays may be labeled or unlabeled. Unlabeled antibodies may be employed in agglutination; labeled antibodies may be employed in a wide variety of assays, employing a wide variety of labels, such as radionuclides, enzymes, fluorescers, enzyme substrates or cofactors, or the like. These techniques are amply defined in the literature and exemplary assays may be found in U.S. Patent Nos. 3,817,834; 3,935,074; 4,233,402 and 4,318,980, as illustrative.

In some techniques it will be useful to label the antigen or fragment thereof, rather than the antibody, and have a competition between labeled antigen and antigen in the sample for antibody. In this situation, it is common to provide kits which have the combination of the labeled antigen or labeled fragment and the antibody in amounts which provide for optimum sensitivity and accuracy. In other situations, it is desirable to have a solid support, where either antigen or antibody is bound. A polyepitopic antigen can serve as a bridge between antibody bound to a support and labeled antibody in the assay medium. Alternatively, one may have a competition between labeled antigen and any antigen in the sample for a limited amount of antibody.

Where the antigen may not be found in a physiological fluid or if found there is not diagnostic of malignancy or the target proliferative disease, then cells will have to be isolated and the cells assayed for the presence of the antigen. For detecting the antigen, the tissue sample may be lysed by conventional methods, e.g., base, detergents, or the like, cellular debris separated by filtration or centrifugation and the filtrate or supernatant isolated and assayed.

For purposes of therapy, either xenogeneic or allogeneic antibodies may be employed, depending upon the nature of the treatment, and whether the foreign antibodies will induce an immune response. The literature has described a number of ways of making human antibodies, where it is found that mouse or other mammalian antibodies are not satisfactory. The antibodies may be used in a wide variety of ways. By employing the appropriate IgG (other than IgG₁), one may induce lysis through the natural complement process. Alternatively, the lysing portion of a toxin may be joined to the antibodies, particularly a Fab fragment. The antibodies may be bound to liposomes for directing the liposomes to the malignant cells to become ingested by the cells by merging of the membranes. Other labels may also be bound to the antibodies, such as radionuclides, fluorescers, enzymes, and the like. By introducing the antibodies in vivo, the antibodies will direct the label to the malignant cell, where the presence of malignancy may be diagnosed or treated.

The formulation of the antibodies will vary widely, depending on the nature of the label, the purpose of the antibodies, the site to which the antibodies are to be directed, and the like. Usually, the antibodies will be formulated in a physiologically acceptable carrier, e.g. saline or phosphate buffered saline, and injected into the host, when possible at the desired site, and when this is not possible, into a circulating system, such as blood.

The antibodies obtained in accordance with this invention can also be used to isolate cells expressing the TGF polypeptides and to remove cells in vitro from a heterogeneous cell population containing cells expressing a TGF polypeptide. Separation can be achieved with a fluorescence activated cell sorter (FACS). This same technique can be used for identifying and isolating cells expressing a TGF polypeptide. For removing cells expressing a TGF polypeptide from a mixture of cells, the subject antibodies may be combined with complement, joined to the lysing fragment (A fragment) of a toxin (see E.P.O. application No. 17,507 and U.K. Patent Application No. 2,034,324) or the cells agglutinated and separated by physical means.

Methods and compositions employing the biologically active polypeptides and oligopeptides of the invention are also afforded for treatment of cancer and other proliferative diseases and for therapies wherein cell growth promotion is beneficial. In particular, compositions are provided employing the oligopeptides of formula III above for the treatment of malifnancies. Further compositions containing biologically active polypeptides of formulas I and II for treatment of cancer and other proliferative diseases and for cell growth promotion application,s e.g., wound healing and ulcer therapy are also provided. These therapeutic compositions comprise effective amounts of the indicated oligopeptides and polypeptides in admixture with pharmaceutically acceptable carriers. In particular, pharmaceutical compositions that contain the oligopeptides and/or polypeptides of the invention as an active ingredient will normally be formulated with an appropriate solid or liquid carrier depending upon the particular mode of administration being used. For instance, parenteral formulations are usually injectable fluids that use pharmaceutically and physiologically acceptable fluids such as physiological saline, balanced salt solutions, or the like as a vehicle. Oral formulations, on the other hand, may be solid, e.g., tablet or capsule, or liquid solutions or suspensions.

In the therapeutic methods of the invention, the oligopeptides and/or polypeptides may be administered to humans in various manners such as orally, intravenously, intramuscularly, intraperitoneally, intranasally, intradermally, and subcutaneously. The particular mode of administration and dosage regimen will be selected by the attending physician taking into account the particulars of the patient, the nature of treatment required, and/or the disease and the disease state involved. For instance, damaged tissue from wounds is usually treated by daily or twice daily doses over a few days to a few weeks; whereas tumor or cancer treatment involves daily or multidaily doses over months or years. The oligopeptide and/or polypeptide therapy of the invention may be combind with other treatments and may be combined with or used in association with other chemotherapeutic or chemopreventive agents for providing therapy against proliferative diseases, neoplasms, or other conditions against which they are effective.

The following examples are offered by way of illustration and not by way of limitation:

Example I

Production, Purification and Characterization of a low molecular weight Human Transforming Growth Factor (htGFs)

A. Experimental Procedures

Source of hTGFs

hTGFs was purified from the serum-free medium conditioned by a human metastatic melanoma line A2058 (Todaro et al. (1980) Proc. Natl. Acad. Sci. USA 77, pp. 5258-5262) derived from a brain metastasis in a 43-year-old man. Cells were grown to 90% confluency in roller bottles containing Dulbecco's modified Eagle's medium (Grand Island Biological Co., 430-2100), supplemented with 10% calf serum (Colorado Serum Co.,) at 37° C. The cells were washed for 1 h with 50 ml of serum-free Waymouth's medium (Grand Island Biological Co., MD 705/1). This and a second collection of supernatant fluid, 24 h later, were discarded. Subsequent collections were made every other day, or every 3rd day, for a 2-week period.

The medium was collected by decantation, stored for up to 24 h at 4° C in the presence of the protease inhibitor phenylmethanesulfonyl fluoride (1μ g/ml), and clarified by continuous flow centrifugation at 32,000 rpm at 4° C. Flow rates of 5 liters/h in the CF-32 continuous flow rotor (Beckman) in the model L5-50 ultracentrifuge (Beckman) were used. The supernatant, after high speed centrifugation, will be referred to as A2058-conditioned medium.

The A2058-conditioned medium was immediately concentrated in the hollow fiber Dialyzer/Concentrator (Model DC10, type H1095-20 cartridge, Amicron Corp.) at 10° C. The concentrate was drained after a 150-fold reduction in volume. The cartridge was washed with 1000 ml of Waymouth's medium. The ultrafiltrate was discarded.

## Purification of hTGFs

### Dialysis and Centrifugation

The combined retentate and cartridgewash after ultrafiltration of A2058-conditioned medium was dialyzed for 60 h against 0.1 M acetic acid in Spectrapor 3 dialysis tubing (Spectrum Medical Industries). The retentate was centrifuged at 100,000 x g for 1 h at 4° C. The pellet was discarded. The supernatant was concentrated by lyophilization and reconstituted in 0.5 ml of 1 M acetic acid/liter of original A2058-conditioned medium.

### Chromatography on Bio-Gel P-10

Following concentration, dialysis, and centrifugation, the supernatant containing hTGF activity was further purified by gel permeation chromatography on a column (2.5 x 85 cm) (420 ml bed volume) of Bio-Gel P-10 (200-400 mesh, Bio-Rad Laboratories). The column was equilibrated with 1 M acetic acid at 22° C. Samples of protein (65-115 mg) in 1 M acetic acid (5 ml) were applied to the column. To ensure a constant flow rate the column effluent was regulated at 12 ml/h with a peristaltic pump. 4.8-ml fractions were collected. Aliquots were lyophilized for subsequent determinations of EGF-competing activity and growth-promoting activity in soft agar. Fractions representing the major portions of a given peak were pooled and concentrated by lyophilization.

### Reverse Phase High Pressure Liquid Chromatography

The final purification of hTGF was achieved by reverse phase HPLC, using the general procedure described in Marquardt et al. (1981) J. of Biol. Chem 256, pp. 6859-6865. All separations were performed on a μBondapak $C_{18}$ column (10-μm particle size, 0.39 x 30 cm, Waters Associates) at a flow rate of 1 ml/min at 40° C. Lyophilized samples were reconstituted in 0.05% (v/v) trifluoroacetic acid in water, adjusted to pH 2 with 10% (v/v) trifluoroacetic acid, and applied through the sample injector to the column which was equilibrated with 0.05% trifluoroacetic acid. The column was then eluted with a linear acetonitrile gradient in 0.045% trifluoroacetic acid. The column effluent was collected in 1.5-ml fractions. Aliquots were lyophilized for subsequent EGF competition and growth stimulation assays. Pools of fractions comprising the major hTGFs activity were concentrated by lyophilization.

hTGFs-containing pools were reconstituted in 0.05% trifluoroacetic acid and rechromatographed on the same column, previously equilibrated with 0.05% trifluoroacetic acid in water. The column was then eluted with a linear 1-propanol gradient in 0.035% trifluoroacetic acid. The column effluent was collected in 1.5-ml fractions. Aliquots were lyophilized for EGF competition and growth stimulation assays.

### SDS-Polyacrylamide Gel Electrophoresis

SDS-polyacrylamide gel electrophoresis was performed as described in Laemmli (1980) Nature (Lond) 227, pp. 680-685. A 15-30% acrylamide gradient slab (140 x 120 x 0.75 mm) was prepared with a 4% stacking gel. The gels were run at 30 V with an electrode buffer containing Tris (0.05 M), glycine (0.38 M), and SDS (0.1%, w/v) until the tracking dye (bromphenol blue) had run off the end of the gel. After electrophoresis, gels were fixed in 50% methanol, 10% acetic acid for 2 h, washed in 5% methanol, 7% acetic acid overnight, and stained with silver (Oakley et al. (1980) Anal. Biochem. 105, pp. 361-363).

Protein Determination

Total protein was determined using bovine serum albumin as a standard. Prior to protein determination, the starting material was dialyzed against phosphate-buffered saline to remove components of the culture medium interfering with the color reaction or lyophilized if samples had been dissolved in volatile acids. Protein was also determined by amino acid analysis. Lyophilized samples were hydrolyzed at 110°C for 24 h in evacuated Pyrex tubes with 0.1 ml of 6 N HCl containing 0.1% liquid phenol, and analyzed witha Durrum D-500 analyzer equipped with a PDP 8/A computing integrator using o-phthalaldehyde for the fluorogenic detection of primary amines (Bensen et al. (1975) Proc. Natl. Acad. Sci. USA 72, pp. 619-622).

Radioreceptor Assay

Purified EGF was labeled with $Na^{125}I$ by a modification of the chloramine-T method as described in DeLarco et al. (1978) Proc. Natl. Acad. Sci. USA 75, pp. 4001-4005. The $^{125}I$-EGF binding assay was performed on subconfluent monolayers of formalin-fixed A431 human carcinoma cells as previously described (in DeLarco et al. (1980) J. of Biol. Chem. 255, pp. 3685-3690). The fixed cells were washed twice with 0.5 ml of binding buffer (Dulbecco's modified Eagle's medium containing 1 mg/ml of bovine serum albumin and 50 mM 2-[bis(2-hydroxyethyl)amino]ethanesulfonic acid, pH 6.8). Competitions were initiated by the addition of 0.2 ml of binding buffer containing 0.4 ng of $^{125}I$-EGF with or without potential inhibitor. After incubation for 1 h at 22°C, the specifically bound $^{125}I$-EGF was determined. The TGF content was expressed by its degree of inhibition of the binding of $^{125}I$-EGF to the EGF receptor. One EGF-competing activity unit is defined as the amount of protein that inhibits the binding of $^{125}I$-EGF to its receptor by 50%.

Soft Agar Growth Assay

The assay for colony growth in soft agar, using normal rat kidney fibroblasts, clone 49F, was performed as reported in Todaro et al. (1980) Proc. Natl. Acad. Sci. USA 77, pp. 5258-5262. Lyophilized samples to be tested were reconstituted in 0.5 ml of Dulbecco's modified Eagle's medium, supplemented with 10% calf serum. 1.5 ml of 0.5% (w/v) agar (Difco) in the supplemented medium and 0.5 ml of supplemented medium containing $2.3 \times 10^4$ cells were added. 2.3 ml of the resultant mixture were pipetted on a 2-ml base layer (0.5% agar in supplemented medium) in 60-mm Petri dishes (Falcon). The cells were incubated at 37°C in a humidified 5% $CO_2$/95% air atmosphere. The assay was read unfixed and unstained at 5 days and at 10-14 days.

B. Results

Source, Concentration, and Initial Fractionation of hTGFs

hTGFs was isolated from serum-free conditioned medium of the highly transformed human metastatic melanoma cell line, A2058. The quantitation of hTGFs was based on two of its properties: the capacity to induce anchorage-independent growth of normal rat kidney fibroblasts in soft agar, and the ability to compete with $^{125}I$-EGF for the EGF receptor sites on A431 human carcinoma cells. A summary of the steps leading to the isolation of hTGFs and its recovery is presented in Table I.

TABLE I

Purification of hTGFs from conditioned medium of human melanoma cells, A2058

| Purification step | Protein[a] recovered mg | EGF-competing activity recovered units[b] | Relative specific activity units/mg | Degree of purification -fold | Recovery % |
|---|---|---|---|---|---|
| 1. A2058-conditioned medium | 1,020 | 4,525 | 4.4 | 1 | 100 |
| 2. Acid-soluble supernatant | 837 | 4,299 | 5.1 | 1 | 95 |
| 3. Bio-Gel P-10 | | | | | |
| Pool P-10-A | 29.7 | 2,077 | 70 | 16 (1) | 45.9 (100) |
| Pool P-10-B | 14.5 | 2,033 | 140 | 32 (1) | 44.9 (100) |
| 4. Bondapak $C_{18}$(acetonitrile) | 0.202 | 1,628 | 8,059 | 1,832 (57) | 36.0 (80.1) |
| 5. Bondapak $C_{18}$(1-propanol) | 0.0015 | 1,476 | 984,000 | 223,636 (6,988) | 32.6 (72.6) |

[a] Total protein was determined using bovine serum albumin as a standard. The quantitation of step 5 hTGFs was based on amino acid analysis. The absolute specific activity of a companion aliquot was found to be $1-1.5 \times 10^6$ units/mg.

[b] One EGF-competing activity unit is defined as the amount of protein that inhibits the binding of $^{125}I$-EGF to its receptor by 50%.

To remove serum proteins, A2058 cells were extensively washed with Waymouth's medium prior to their culture in serum-free medium. The supernatant fluids were collected every other day for a 2-week period. Culture conditions were such that at the end of the culture period more than 90% of the cells were still viable and attached as monolayers. The initial clarified A2058-conditioned medium of 136 liters, containing 1.02 g of total protein and 4525 units of EGF-competing activity, was concentrated to about 900

14

ml using a hollow fiber concentrator with cartridges of 5000 molecular weight cutoff. The total EGF-competing activity was retained and a recovery above 95% was obtained.

Dialysis of the concentrated A2058-conditioned medium against acetic acid and subsequent centrifugation resulted in 95% recovery of the initial total EGF-competing activity. 18% of the protein was acid-insoluble and was discarded. The acid-soluble, partially purified hTGF was subjected to gel permeation chromatography on Bio-Gel P-10. The column was eluted with 1 M acetic acid. The bulk of the contaminating protein was eluted in the exclusion volume of the column and was well separated from the EGF-competing activity and growth-stimulating activity. Two peaks of activity were found to be well resolved from each other. Fractions with both EGF-competing and growth-stimulating activity (P-10-A and P-10-B) had apparent molecular weights of 10,500 and 6,800, respectively. Fractions having only one of the two activities were not observed. hTGF-containing fractions were pooled as indicated, lyophilized, and further purified. The larger molecular weight TGF eluted from the column in a broad peak (P-10-A0 and appeared to be associated with polypeptides of different sizes. P-10-A contained 46% of the initial EGF-competing activity. The small molecular weight TGF eluted from the column in a sharp peak (P-10-B) and represented 45% of the initial total EGF-competing activity. The cumulative yield of total input EGF-competing activity from step 1 through the gel permeation chromatography step was 91% (Table I). hTGF was eluted as two distinct major peaks that varied quantitatively from one preparation to another. In some preparations of A2058-conditioned medium essentially all the growth-promoting activity was in the hTGFs region.

## Purification of hTGFs

hTGFs was further purified by reverse phase PHLC. Pool P-10-B, after gel permeation chromatography of the acid-soluble EGF-competing activity of concentrated A2068-conditioned medium on Bio-Gel P-10, was reconstituted in 0.05% trifluoroacetic acid in water, and then chromatographed on a $\mu$Bondapak $C_{18}$ column. EGF-competing and growth-stimulating activities in soft agar of individual fractions were determined. hTGFs was well separated from the bulk of contaminating protein which eluted at higher concentrations of organic solvent. Fractions containing hTGFs were pooled, lyophilized, and taken for rechromatography. A 57-fold purification of hTGFs after gel permeation chromatography was obtained. 80% of the initial EGF-competing activity in pool P-10-B was recovered (Table I).

Rechromatography of the hTGFs-containing fractions on $\mu$Bondapak $C_{18}$ support was chosen for the final purification step since only relatively small losses of EGF-competing activity were observed on these columns. In order to obtain a distinct separation of hTGFs from impurities, it was necessary to use a shallow linear 1-propanol gradient in 0.035% trifluoroacetic acid. The bulk of contaminating peptide material was separated from a well defined peak of activity. EGF-competing and growth-stimulating activities copurified with a distinct absorbance peak at 13% 1-propanol. Fractions containing hTGFs were pooled and further analyzed. The purification of hTGFs was approximately 7000-fold after gel permeation chromatography with a yield of 33% of the initial total EGF-competing activity. The overall recovery of hTGFs from step 3 through the final reverse phase HPLC step was 73%, and the recovery range per step was 80-100% (Table I).

## Characterization of hTGFs

The purity of the final hTGFs preparation was determined by analytical SDS-polyacrylamide gel electrophoresis. The gel was stained with silver. One major polypeptide band, with an apparent $M_r = 7400$, was observed. The same pattern was obtained when samples were electrophoresed under nonreducing conditions indicating that TGF is a single chain molecule.

The receptor reactivity of hTGFs was compared with EGF in the radio-receptor assay. The quantitation of hTGFs was based on amino acid analysis of a companion aliquot. Both hTGFs and EGF competed and $^{125}$I-EGF for the EGF receptor sites of A431 human carcinoma cells as shown in Fig. 3A. The specific EGF-competing activity of hTGFs was found to $1-1.5 \times 10^6$ units/mg; 1.1 ng of hTGFs or EGF were required to inhibit EGF binding by 50%

hTGFs enabled normal anchorage-dependent rat kidney cells, clone 49F, to grow in soft agar. The half-maximal response of hTGFs in soft agar was reached with 1 EGF-competing unit, or 1.1 ng of hTGFs, whereas EGF does not stimulate growth of these cells in soft agar even when tested with up to $10\mu$ g.

## Example II

Larger Scale Production, Purification and Amino Acid Sequencing of Human (hTGF), Rat (rTGF), and Mouse (mTGF) Transforming Growth Factors

A. Experimental Procedures

Source of TGF

rTGF, mTGF and hTGF were purified from the serum-free medium conditioned by Fisher rat embryo fibroblasts, FRE CII0, a subclone of FRE 3A (Sacks et al. (1979) Virology 97, pp. 231-240), nonproductively transformed by Snyder-Theilen feline sarcoma virus (Snyder et al. (1969) Nature (Lond) 221, pp. 1074-1075), a Moloney murine sarcoma virus-transformed 3T3 cell line, 3BII-IC (Bassin et al. (1970) Int. J. Cancer 6, pp. 95-107), and two human metastatic melanoma lines, A2058 (see Example I), and A375 (Girad et al. (1973) J. Natl. Cancer Inst. 51, pp. 1417-1423), respectively. Cells were grown in 2-liter plastic roller bottles containing Dulbecco's modified Eagle's medium supplemented with 10% calf serum and subsequently maintained in serum-free Waymouth's medium as described in DeLarco et al. (1978) Proc. Natl. Acad. Sci. USA 75, pp. 4001-4005. Serum-free conditioned medium was collected every 24 h, for a 3-day period, clarified by continuous flow centrifugation, and the supernatant concentrated (Marquardt et al. (1980) J. of Biol. Chem. 255, pp. 9177-9181). The concentrate of conditioned medium was the starting material for the purification of TGFs.

Purification of TGF

The TGFs were prepared essentially by methods previously described in Example I for the purification of the melanoma-derived hTGF. The retentate after ultrafiltration of conditioned medium was dialyzed against 0.1 M acetic acid, and the supernatant, after centrifugation, concentrated by lyophilization and reconstituted in 1 M acetic acid for subsequent gel permeation chromatography on a column (2.5 x 85 cm) of Bio-Gel P-10 (200-400 mesh, Bio-Rad Laboratories). The column was equilibrated with 1 M acetic acid. Fractions comprising the major EGF-competing activity with an apparent molecular weight of approximately 7,000 were pooled and lyophilized.

The final purification of rTGF, mTGF, and hTGF was achieved by reverse phase HPLC using the chromatography system described in Example I. The separations were performed on a $\mu$Bondapak C$_{18}$ column (10 $\mu$m particle size, 0.39 x 30 cm, Waters Associates). The mobile phase was 0.05% trifluoroacetic acid and the mobile phase modifier was acetonitrile containing 0.045% trifluoroacetic acid. The concentration of acetonitrile was increased linearly (0.083%/min) during 2 h at a flow rate of 1 ml/min at 4°C for elution of peptides. TGF-containing pools were lyophilized and reconstituted in 0.05% trifluoroacetic acid and rechromatographed on the same column, using as the mobile phase modifier 1-propanol containing 0.035% trifluoroacetic acid. The 1-propanol concentration was increased linearly (0.05%/min) during 2 h at a flow rate of 1 ml/min at 40°C. Pools of fractions comprising the major EGF-competing activity were lyophilized.

Assay for TGF

TGF was quantitated in a radioreceptor assay based on receptor crossreactivity with mouse submaxillary gland epidermal growth factor (mEGF). Purified mEGF was labeled with Na$^{125}$I by a modification of the chloramine-T method as described in Example I. The $^{125}$I-EGF binding assay was performed on formalin-fixed A431 human carcinoma cells, 8 x 10$^3$, in Micro Test II plates (Falcon). The concentration of TGF was expressed in mEGF ng equivalents/ml and was based on the amount of TGF required to produce equal inhibition of $^{125}$I-EGF binding to A431 cells as a known amount of unlabeled mEGF.

Amino Acid Sequence Determination of TGF

For amino acid sequence analysis, rTGF (3 $\mu$g) was reduced with dithiothreitol (20 mM) in 100 1 of Tris-HCl buffer (0.4 M) containing guanidine-HCl (6 M) and Na$_2$-EDTA (0.1%), pH 8.5, for 2 h at 50°C, and subsequently S-carboxamidomethylated with iodoacetamide (45 mM) for 30 min at 22°C. The S-carboxamidomethylated rTGF was de-salted on a $\mu$Bondapak C$_{18}$ column. Peptide was eluted with a gradient of aqueous acetonitrile containing 0.045% trifluoroacetic acid. The concentration of acetonitrile was increased linearly (1%/min) during 1 h at a flow rate of 1 ml/min at 40°C.

Automated sequence analyses (Edman et al. (1967) Eur. J. Biochem. 1, pp. 80-91) of S-carboxamidomethylated rTGF and unmodified mTGF and hTGF were performed with a gas-liquid solid phase microsequenator (Hewick et al. (1981) J. of Biol. Chem. 256, pp. 7990-7997). Sequenator fractions were analyzed by reverse phase HPLC (Hunkapiller et al. (1983) Science 219, pp. 650-659).

B. Results

Purification of TRGF

Purified preparations of a small molecular weight rTGF, mTGF and hTGF were obtained from the conditioned medium of retrovirus-transformed rat and mouse fibroblasts and two human melanoma cell lines, respectively. The purification was achieved by gel permeation chromatography of the acid-soluble EGF-competing activity on Bio-Gel P-10 in 1 M acetic acid, followed by reverse phase HPLC on $\mu$Bondapak $C_{18}$ support using sequentially a linear gradient of aqueous acetonitrile and subsequently 1-propanol containing 0.035% trifluoroacetic acid. The elution patterns of the final purification step of rTGF, mTGF and hTGF show that EGF-competing activity co-purified with a distinct absorbance peak, and was effectively separated from contaminating UV-absorbing material. The major protein peak in rTGF, mTGF and hTGF preparations eluted from a $\mu$Bondapak $C_{18}$ column under standard conditions between 48 and 55 min.

Gel permeation chromatography on Bio-Gel P-10 provided a separation of the small molecular weight TGFs from larger molecular weight TGFs and reduced the load of protein applied to a $\mu$Bondapak $C_{18}$ column in the following purification step. The small molecuar weight TGFs represented 45 to 80% of the initial total EGF-competing activity. Reverse phase HPLC of TGFs on $\mu$Bondapak $C_{18}$ support in the following two purification steps was very efficient, each giving in a typical preparation a recovery range of 80 to 100% per step. The final recovery of the small molecular weight TGFs was approximately 70%, based on the maximal total EGF-competing activity detected during the course of the purification. The average yield of purified rTGF was 90 ng/liter, of mTGF 50 ng/liter and of hTGF 10 ng/liter of conditioned medium. This calculation is based on the specific activity determined for isolated TGFs and on the assumption that the EGF-competing activity measured in the radioreceptor assay reflects levels of total large and small molecular weight TGFs only. No immunoreactive mEGF was detected in conditioned medium.

Purity of TGF

The purity of rTGF, mTGF and hTGF, suggested by the chromatographic elution profiles, was assessed in the EGF radioreceptor assay and by amino acid sequence analysis. rTGF, mTGF and hTGF competed with $^{125}$I-EGF for the EGF receptor sites on A431 human carcinoma cells and were qualitatively and quantitatively nearly indistinguishable from mEGF. Hence, the final TGF preparations were believed to be highly purified and essentially at homogeneity. A single amino-terminal sequence was determined by automated Edman degradation for rTGF, mTGF and hTGF. Any unblocked minor peptide sequence present at >5% could have been detected by the methods used. The homogeneity of hTGF was confirmed in addition by analytical sodium dodecyl sulfate-polyacrylamide gel electrophoresis. The purified preparation gave one major polypeptide band.

Amino Acid Sequencing of TGF

The complete sequencing of the rTGF, mTGF and hTGF was accomplished and the amino acid sequences for the three polypeptides are given below. It will be noted from the sequences reported that rTGF and mTGF are identical in chemical make-up and further that substantial homology exists between the murine TGFs and hTGF with different amino acid residues occurring at only positions 7, 15, 23 and 38 in the sequences.

```
(1)   rTGF
                  5                      10                     15
      Val-Val-Ser-His-Phe-Asn-Lys-Cys-Pro-Asp-Ser-His-Thr-Gln-Tyr-
                  20                     25                     30
      Cys-Phe-His-Gly-Thr-Cys-Arg-Phe-Leu-Val-Gln-Glu-Glu-Lys-Pro-
                  35                     40                     45
      Ala-Cys-Val-Cys-His-Ser-Gly-Tyr-Val-Gly-Val-Arg-Cys-Glu-His-
                  50
      Ala-Asp-Leu-Leu-Ala
```

(2) mTGF

```
                  5                        10                        15
Val-Val-Ser-His-Phe-Asn-Lys-Cys-Pro-Asp-Ser-His-Thr-Gln-Tyr-
                 20                        25                        30
Cys-Phe-His-Gly-Thr-Cys-Arg-Phe-Leu-Val-Gln-Glu-Glu-Lys-Pro-
                 35                        40                        45
Ala-Cys-Val-Cys-His-Ser-Gly-Tyr-Val-Gly-Val-Arg-Cys-Glu-His-
                 50
Ala-Asp-Leu-Leu-Ala
```

(3) hTGF

```
                  5                        10                        15
Val-Val-Ser-His-Phe-Asn-Asp-Cys-Pro-Asp-Ser-His-Thr-Gln-Phe-
                 20                        25                        30
Cys-Phe-His-Gly-Thr-Cys-Arg-Ser-Leu-Val-Gln-Glu-Glu-Lys-Pro-
                 35                        40                        45
Ala-Cys-Val-Cys-His-Ser-Gly-Phe-Val-Gly-Val-Arg-Cys-0Glu-His-
                 50
Ala-Asp-Leu-Leu-Ala
```

The sequence shown for hTGF has subsequently been found to be in error: the correct sequence is on file at the European Patent Office.

Example III

Production of TGF in vivo and its Isolation

Tumor cell lines (1 x $10^6$) known to produce TGFs (human melanoma and transformed rat) were inoculated into athymic "nude" mice and tumors were allowed to develop. The urine from the tumor-carrying mice was collected and analyzed for the presence of TGF using the isolation procedure and analytical techniques given in Example I above. TGF was detected in the urine of the tumor carrying mice which has the same size and elution properties on HPLC as does the cell culture derived TGF which is described in Examples I and II above. Further, using the procedures described in Example I above, the TGF present in the mouse urine was found to have the characteristic TGF biological properties, in that it stimulates anchorage-independent growth of cells and binds to the EGF receptor. Subsequently, the tumors were removed from the tumor-carrying mice and the urine of the mice after tumor removal was tested for the presence of TGF using the above mentioned procedures. In this case, no TGF was found having the characteristic elution properties on HPLC or TGF-like biological activity. These results demonstrate that tumor cells produce TGF in whole animals as well as cell cultures and that TGF can be detected in and isolated from body fluids using the process of the invention. Similar experiments were also performed with rats having chemical carcinogen-induced tumors and they were found to have TGF in their urine, based on the biological and biochemical properties listed above while untreated rats did not.

Example IV

Inhibition of Retroviral Transformed Cell Growth In Vitro with Antibodies to Antigenic TGF Oligopeptide

An oligopeptide having the following amino acid sequence (which corresponds to amino acid sequences 34 through 50 of rat TGF):

```
Cys-His-Ser-Gly-Tyr-Val-Gly-Val-Arg-Cys-His-Ala-

Asp-Leu-Leu-Ala
```

was synthesized using the solid-phase technique of Ohgak et al. (1983) Journal of Immunol. Meth. 57, pp.

171-184. This oligopeptide was then coupled to keyhole limpet hemocyanin in accordance with the procedure of Baron et al., (1982) Cell 28, pp. 395-404, and used to immunize rabbits (Baron et al. (1982), Cell 28, pp. 395-404) and sheep (Lerner (1982) Nature 299, pp. 592-596). Antisera were assayed against peptide by a peroxidase-linked immunoassay (Kirkegaard and Perry Laboratories, Gaithersberg, MD) and against homogeneous rat TGF (purified according to Example II above), by immunoprecipitation (Bister et al., (1980) J. Virol. 36, pp. 617-621) and Western blotting techniques (Burnett (1981) Analyt. Biochem. 112, pp. 195-203). Binding of $^{125}$I-labeled rat TGF and mouse EGF (Bethesda Research Labs, Bethesda, MD) to A431 cells grown in 96-well microtiter plates was as described in Pruss et al. (1977) Proc. Natl. Acad. Sci. USA 74, pp. 3918-3921.

Antisera prepared in one rabbit and two sheep reacted with the corresponding peptide in peroxidase-linked immunoassays in titers of at least $10^4$. Reactions of the rabbit antiserum with rat TGF were documented by immunoprecipitation and confirmed by Western blotting. The antipeptide antisera did not immunoprecipitate iodinated mouse EGF in this study.

Human epidermoid A431 cells have in excess of $10^6$ EGF receptors per cell (Fabricant et al. (1977) Proc. Natl. Acad. Sci. USA 74, pp. 565-569). TGF competes with EGF for binding to these receptors. Binding of $^{125}$I-labeled rat TGF to these cells was blocked by an excess of unlabeled EGF and by antiserum to peptide. A blocking effect of antiserum on TGF binding was observed even if the antibody-TGF complex as not removed from the medium surrounding the A431 cells by S. aureus protein A-facilitated immunoprecipitation. Blocking by the antibody was a result of interaction with the TGF molecule rather than with the receptor, since antisera did not react in immunoprecipitation assays with purified iodinated EGF receptor. As anticipated from immunoprecipitation data, antiserum to peptide did not interfere with binding of murine EGF to A431 cells.

With the availability of antisera that blocked cellular binding of TGF but not EGF, it was possible to test whether TGF functions by an autocrine mechanism is stimulating the growth of malignant cells and whether antibody can thus inhibit such growth in vitro. Consequently, untransformed normal rat kidney cells (NRK) and a variety of retroviral cell lines were plated at low densitites (500 to 2,000 cells per dish) in serum-containing medium and after a brief period of adherence were switched to serum-free medium. Sheep or rabbit antibodies to TGF peptide or to various irrelevant non-cross-reacting peptides were added to the medium, and the effect on cell growth, on microscopic and macroscopic colony formation, and on colonial morphology was observed. All antibodies were affinity purified on peptide columns, extensively dialyzed, concentrated, and reconstituted to an antipeptide titer of $10^3$ to $10^4$. The results are provided in Table II below.

TABLE II

| EFFECT OF ANTIPEPTIDE ANTIBODIES ON GROWTH OF CELLS IN VITRO | | | | | | | |
|---|---|---|---|---|---|---|---|
| Antibody | Source | Volume (1) | Other Addition | % Inhibition of Colony Formation by | | | |
| | | | | NRK cells | CI0 cells | Ki-NRK | src-3T3 |
| Anti-TGF oligopeptide | Rabbit | 5 | --- | 0 | 85 | NT | 53 |
| | | 5 | TGF peptide | NT | 36 | NT | 6 |
| Anti-TGF oligopeptide | Sheep | 2 | | 0 | 50 | 49 | NT |
| | | 5 | | 0 | 68 | 85 | 77 |
| | | 10 | | 0 | 79 | 73 | 100 |
| | | 5 | TGF peptide | NT | 9 | 20 | 43 |
| | | 5 | Irrelevant peptide | NT | 85 | NT | NT |
| Anti-heteropeptides (4) | Sheep & rabbit | | | 0 | <15 | ≦5 | 0 |

As seen in Table II, above, neither rabbit nor sheep anti-TGF oligopeptide inhibited colony formation by NRK cells but within 48 hours inhibited growth of Kirsten-Transformed NRK cells, feline-sarcoma-virus-transformed rat embryo fibroblasts (CLI0 cells), and Rous-sarcoma-virus-transformed 3T3 cells. CLI0 cells were known to be prolific producers of TGF (Marquardt et al. (1983) Proc. Natl. Acad. Sci. USA 80, pp. 4684-4688). The few surviving colonies in anti-TGF antibody-treated cells tended to be smaller and lacked the robust appearance of normal colonies. Non-adherent unlysed cells floated free in the medium. This inhibition was partially reversed when TGF peptide, but not non-cross-reacting irrelevant peptide, was added concomitantly with antibody to TGF. Rabbit and sheep antibodies to four different non-cross-reacting peptides had no effect on growth of colonies of NRK or retroviral transformed cell lines. Replacement of antibody-containing medium by fresh antibody-free medium after 72 hours failed to reverse the inhibition of colony formation, but the surviving colonies grew vigorously.

Example V

Synthesis and Characterization of rat TGF

The chemical synthesis of rat TGF (rTGF), having the chemical formula given in Example II, was performed manually by the stepwise solid-phase approach according to the general principles described by Merrifield (1963) J. Amer. Chem. Soc. 85, pp. 2149-2156. The differential acid-labile protecting group strategy was adopted for this synthesis with the conventional combination of tertbutyl-oxycarbonyl for N-amino terminus and benzyl alcohol derivatives for the side chains. A more acid stable benzyl ester linkage that anchored protected amino acids to the polymeric support was used to minimize loss of peptides during the repetitive acid treatments (Mitchell et al. (1976) J. Amer. Chem. Soc. 92, pp. 7357-7362). Complete deprotection and removal of peptide from the resin was by the low-high HP method of Tam et al. (1982) Tetrahedron Lett. 23, pp. 4435-4438, which differed from the conventional HF deprotection method and removed benzyl protecting groups by the $S_N2$ mechanism in dilute HF solution to minimize serious side reactions due to carbocations generated in the conventional $S_N1$ deprotection method. Furthermore, it is also designed to reduce many cysteinyl side reactions that often hamper the synthesis of proteins containing multiple disulfide linkages.

After HF treatment and prior to any purification, the crude and reduced synthetic rTGF was oxidized and regenerated by the mixed disulfide method in the presence of a combination of reduced and oxidized glutathione (Ahmed et al. (1975) J. Biol. Chem. 250, pp. 8477-8482). This avoided the formation of polymeric materials during purification. The regenerated, crude rTGF-I contained 40-50% of EGF-radioreceptor and tyrosine-specific protein kinase activities when compared to the natural rTGF-I. Crude synthetic rTGF-I was purified to homogeneity in three steps: (1) gel filtration on a Bio-Gel P-10 column; (2) ion-exchange chromatography on a CM-Sephadex column; and (3) preparative high pressure liquid chromatography (HPLC) on a C-18 reverse phase column. An overall yield, based on starting loading of Ala to resin, was 31%.

Under reducing or nonreducing conditions, the purified synthetic rTGF-I was found to give a single band with an apparent M.W. of 7000 on SDS-PAGE electrophoresis. Amino acid analysis by 6N HCl and enzymatic hydrolysis provided the expected theoretical molar ratio of the proposed sequence. No free thiol was detected by Ellman's method of sulhydryl determination on synthetic rTGF, but upon thiolytic reduction, the expected theoretical value of six cysteines was obtained. These findings support the conclusion that synthetic rTGF is a single chain polypeptide containing six cysteines in disulfide linkages, which is in agreement with the expected chemical properties of the natural rTGF. Additionally, synthetic rTGF coeluted with the natural rTGF as a single symmetrical peak in C-18 reverse phase HPLC.

Synthetic rTGF prepared in accordance with this Example was compared with natural rTGF in three assays for biological properties of the putative transforming growth factor. In the mitogen assay, the stimulation of growth of serum-deprived normal rat kidney cells by rTGF was measured by the incorporation of $^{125}$I-Iododeoxyuridine. In the soft agar assay in the presence of fetal bovine serum and a second TGF, TGF-beta, the morphological and phenotypic alterations by rTGF could be quantitated by colony formation in soft agar. The latter transforming assay has been shown to correlate well with tumorigenicity (Stoker et al. (1968) Int. J. Cancer 3, pp. 683-693). Fetal bovine serum or TGF-beta alone does not induce transformation of NRK cells in culture. Similarly, TGF, natural or synthetic, does not produce such an effect in the absence of TGF-beta. Both synthetic and natural rTGF displayed similar dose response curves and half maximal activities in these two assays.

Since rTGF competes with mEGF for the binding of EGF receptors on cellular membranes, synthetic rTGF was compared with the natural rTGF for binding on A431 human carcinoma cells. Again, the response and activities of the natural synthetic rTGF were found to be indistringuishable from each other. The concentration required for 50% inhibition of $^{125}$I-EGF binding was found to be 3.5 and 4.1 nM for the natural and synthetic rTGF respectively. A consequence of TGF or EGF binding to the EGF membrane receptors is the stimulation of phosphorylation of tyrosine residues of synthetic peptides or endogenous substrates (Pike et al. (1982) J. Biol. Chem. 257, pp. 14628-14631). Synthetic rTGF was found to stimulate the phosphorylation of the synthetic angiotensinyl peptide substrate with a half maximal activity of 0.3 nM, an activity

EP 0 132 021 B1

comparable to the value for natural rTGF, reported by Reynold et al. (1981) Nature 292, pp. 259-261.

**Claims**

1. Biologically active polypeptides containing at least one peptide sequence of the formula:

$$-Cys_1-(AA)_a-Cys_2-(AA)_b-Cys_3-(AA)_c-Cys_4-$$
$$AA-Cys_5-(AA)_d-Cys_6-$$

wherein each AA is an amino acid residue selected from Val, Ser, His, Phe, Asn, Lys, Asp, Thr, Gln, Arg, Leu, Glu, Pro, Ala, Gly, Trp and Tyr; a is 7, b is 4 or 5, c is 10, and d is 8; there are disulfide bridges between $Cys_1$ and $Cys_3$, $Cys_2$ and $Cys_4$, and $Cys_5$ and $Cys_6$, respectively; and with the proviso that the polypeptide contains at least ten different amino acids AA and with the proviso that no amino acid AA is repeated more than three times consecutively.

2. The biologically active polypeptide according to claim 1, wherein b is 4.

3. Biologically active polypeptides or oligomers thereof of the formula:

$$(AA)_n-Cys_1-(AA)_m-Cys_2-(AA)_o-Cys_3-(AA)_p-$$
$$Cys_4-AA-Cys_5-(AA)_q-Cys_6-(AA)_r$$

wherein AA is an amino acid residue selected from Val, Ser, His, Phe, Asn, Lys, Asp, Thr, Gln, Arg, Leu, Glu, Pro, Ala, Gly, Trp and Tyr; and n is an integer of from 4 to 10, m is 7, o is 4 or 5, p is 10, q is 8 and r is an integer of from 6 to 12; there are disulfide bridges between $Cys_1$ and $Cys_3$, $Cys_2$ and $Cys_4$, and $Cys_5$ and $Cys_6$, respectively; and with the proviso that the polypeptide contains at least ten different amino acids AA and with the proviso that no amino acid AA is repeated more than three times consecutively.

4. The biologically active polypeptide according to claim 3, wherein o is 4 and n and r are 7.

5. Biologically active polypeptides containing at least one peptide sequence of the formula:

$$-Cys_1-(AA)_a-Cys_2-(AA)_b-Cys_3-(AA)_c-Cys_4-$$
$$AA-Cys_5(AA)_d-Cys_6-$$

wherein AA is an amino acid residue selected from Val, Ser, His, Phe, Ile, Met, Asn, Lys, Asp, Thr, Gln, Arg, Leu, Glu, Pro, Ala, Gly, Trp and Tyr; a is 7, b is 4, c is 10, and d is 8; there are disulfide bridges between $Cys_1$ and $Cys_3$, $Cys_2$ and $Cys_4$, and $Cys_5$ and $Cys_6$, respectively; and with the proviso that the polypeptide contains at least ten different amino acids AA and with the proviso that no amino acid AA is repeated more than three times consecutively.

6. Biologically active polypeptides or oligomers thereof of the formula:

$$(AA)_n-Cys_1-(AA)_m-Cys_2-(AA)_o-Cys_3-(AA)_p-$$
$$Cys_4-AA-Cys_5-(AA)_q-Cys_6-(AA)_r$$

21

wherein AA is an amino acid residue selected from Val, Ser, Ile, Met, His, Phe, Asn, Lys, Asp, Thr, Gln, Arg, Leu, Glu, Pro, Ala, Gly, Trp and Tyr; n is an integer of from 4 to 10, m is 7, o is 4, p is 10, q is 8 and r is an integer of from 6 to 12; there are disulfide bridges between $Cys_1$ and $Cys_3$, $Cys_2$ and $Cys_4$, and $Cys_5$ and $Cys_6$, respectively; and with the proviso that the polypeptide contains at least ten different amino acids AA and with the proviso that no amino acid AA is repeated more than three times consecutively.

7. A polypeptide transforming growth factor or oligomers thereof of the formula:

```
Val-Val-Ser-His-Phe-Asn-R-Cys₁-Pro-Asp-Ser-His-Thr-
Gln-R'-Cys₂-Phe-His-Gly-Thr-Cys₃-Arg-R''-Leu-Val-
Gln-Glu-Glu-Lys-Pro-Ala-Cys₄-Val-Cys₅-His-Ser-Gly-
R'''-Val-Gly-Val-Arg-Cys₆-Glu-His-Ala-Asp-Leu-Leu-
Ala
```

wherein R is Asp or Lys; R' is Phe or Tyr; R'' is Ser or Phe; R''' is Phe or Tyr; and there are disulphide bridges between $Cys_1$ and $Cys_3$, $Cys_2$ and $Cys_4$, and $Cys_5$ and $Cys_6$, respectively.

8. The polypeptide according to claim 7, wherein R is Asp, R' is Phe, R'' is Ser and R''' is Phe.

9. The polypeptide according to claim 7, wherein R is Lys, R' is Tyr, R'' is Phe and R''' is Tyr.

10. A polypeptide growth factor containing one or more of the following peptide fragments:

A. Val-Val-Ser-His-Phe-Asn-Lys-Cys-Pro-Asp-Ser-His-Thr-Gln-Tyr-Cys-Phe-His-Gly-Thr-Cys

B. Val-Val-Ser-His-Phe-Asn-Asp-Cys-Pro-Asp-Ser-His-Thr-Gln-Phe-Cys-Phe-His-Gly-Thr-Cys

C. Leu-Val-Gln-Glu-Glu-Lys-Pro-Ala-Cys-Val-Cys-His-Ser-Gly, and

D. Cys-His-Ser-Gly-Tyr-Val-Gly-Val-Arg-Cys-Glu-His-Ala-Asp-Leu-Leu-Ala

11. An antigenic oligopeptide selected from the class consisting of:

A. Val-Val-Ser-His-Phe-Asn-Asp-Cys-Pro-Asp-His-Thr

B. Val-Val-Ser-His-Phe-Asn-Lys-Cys-Pro-Asp-His-Thr

22

EP 0 132 021 B1

    C.     Arg-Phe-Leu-Val-Gln-Glu-Glu-Lys-Pro-Ala

    D.     Arg-Tyr-Leu-Val-Gln-Glu-Glu-Lys-Pro-Ala

    E.     Cys-His-Ser-Gly-Phe-Val-Gly-Val-Arg-Cys-Glu-
             His-Ala-Asp-Leu-Leu-Ala

    F.     Cys-His-Ser-Gly-Tyr-Val-Gly-Val-Arg-Cys-Glu-
             His-Ala-Asp-Leu-Leu-Ala

**12.** An oligopeptide having an ability to bind to cellular growth factor receptors and having the formula:
$(AA)_x$-Cys-$(AA)_y$-Gly-R-$(AA)_z$-Arg-Cys-$(AA)_{z'}$,
wherein R is Phe or Tyr and AA is an amino acid residue selected from Val, Asn, His, Ser, Lys, Ile, Gly, Leu, Asp, Asn, Cys, Thr, Ala, Tyr, Pro, Glu, Gln, and Arg, and x is 0 or an integer of from 1 to 6, y is 2, z is 3 and z' is 0 or an integer of from 1 to 6.

**13.** The oligopeptide according to claim 12, wherein x and z' are 0 and AA is an amino acid residue selected from Val, His, Ser, Ile, Gly, and Asp.

**14.** A biologically active oligopeptide of the formula:
$(AA)_x$-Cys-$(AA)_{\sigma'}$-Gly-$(AA)_{\sigma'}$-Gly-$(AA)_{\sigma'}$-Cys-$(AA)_z$,
wherein AA is an amino acid residue selected from Phe and the amino acid residues designated by AA in claim 12 and the subscripts x and z' are as defined in claim 12.

**15.** The oligopeptide according to claim 13, selected from the class consisting of:
    (1) Cys-His-Ser-Gly-Tyr-Val-Gly-Val-Arg-Cys and
    (2) Cys-His-Ser-Gly-Phe-Val-Gly-Val-Arg-Cys

**16.** Antibodies raised to the polypeptide growth factors of claims 7 and 10 or the antigenic oligopeptides of claim 11.

**17.** Antibodies according to claim 16, labeled with a label capable of providing a detectable signal.

**18.** Antibodies according to claim 16, labeled with a cytotoxic agent.

**19.** Antibodies raised to transforming growth factor polypeptides obtained from body fluids of transforming growth factor-producing, tumor-bearing mammals, said polypeptides being in accordance with any of claims 1 to 6.

**20.** A process for the isolation of a homogenous transforming growth factor polypeptide, being a polypeptide in accordance with any of claims 1 to 6, from an aqueous medium containing said transforming growth factor polypeptide in impure form by the process steps comprising:
    (1) dialyzing the aqueous medium containing the transforming growth factor in impure form against aqueous acetic acid to afford a solvent phase containing transforming growth factor polypeptide which phase is concentrated and optionally clarified,
    (2) reconstituting the concentrated solvent phase of step (1) with aqueous acetic acid and subjecting the reconstituted solution to gel permeation chromatography by applying reconstituted solution to a gel permeation chromatography column conditioned with aqueous acetic acid and eluting with aqueous acetic acid to obtain selected fractions of eluate containing transforming growth factor polypeptide in an enhanced state of purity, said selected fractions being combined and concentrated, to afford a partially purified, transforming growth factor polypeptide-containing product,

23

EP 0 132 021 B1

(3) subjecting the partially purified, transforming growth factor polypeptide-containing product of step (2) to sequential reverse phase high pressure chromatography by passing said product after reconstitution in aqueous trifluoroacetic acid, through one or more hydrocarbon bonded silica matrix columns, which have been equilibrated with aqueous trifluoroacetic acid, under high pressure liquid chromatography conditions, the initial column elution being performed using a linear acetonitrile gradient in aqueous trifluoroacetic acid and the subsequent column elution, which is carried out on the combined, transforming growth factor polypeptide-containing fractions of the initial high pressure chromatography step, being performed using a linear 1-propanol gradient in aqueous trifluoroacetic acid, said 1-propanol gradient being increased in sufficiently small 1-propanol concentration increments to afford the transforming growth factor polypeptide as a single distinct peak in the state of a homogeneous polypeptide.

21. The process according to claim 20, wherein the aqueous medium containing said transforming growth factor polypeptide in impure form is a body fluid containing transforming growth factor or an aqueous medium conditioned with a transforming growth factor-producing cell line.

22. A homogeneous transforming growth factor polypeptide obtained by the process of claim 21.

23. The homogeneous transforming growth factor polypeptide according to claim 22, wherein the homogeneous polypeptide is a human transforming growth factor polypeptide.

24. Antibodies raised to the homogeneous transforming growth factor polypeptides of claim 22.

25. Antibodies according to claim 19 or claim 24, labeled with a label capable of providing a detectable signal.

26. Antibodies according to claim 19 or claim 24, labeled with a cytotoxic agent.

27. An in vitro method for detecting malignancy in a human host which comprises contacting cells or body fluids from said human host with an antibody of claim 16, claim 19 or claim 24 and determining the level of binding of said antibody to said cells or cellular products in said body fluid as a diagnostic of a host carrying malignancy.

28. A composition for treatment of cancer or other proliferative disease which comprises a therapeutic amount of a polypeptide of claim 7 or claim 10 or an oligopeptide of claim 12 or claim 14 together with a pharmaceutically acceptable carrier therefor.

29. A composition for treatment and/or repair of cell or tissue damage which comprises an effective amount of a polypeptide of claim 1, claim 3, claim 5 or claim 6 together with a pharmaceutically acceptable carrier therefor.

30. A biologically active peptide according to any one of claims 1, 3, 5 or 6 for treating and/or repairing cell or tissue damage.

31. A polypeptide according to claim 7 for treating and/or repairing cell or tissue damage.

32. An antibody according to any one of claims 16, 19 or 24 for detecting malignancy in a human host.

**Revendications**

1. Polypeptides biologiquement actifs renfermant au moins une séquence peptidique de formule:
   $-Cys_1-(AA)_a-Cys_2-(AA)_b-Cys_3-(AA)_c-Cys_4-AA-Cys_5-(AA)_d-Cys_6-$
   dans laquelle chaque AA est un résidu d'acide aminé choisi parmi Val, Ser, His, Phe, Asn, Lys, Asp, Thr, Gln, Arg, Leu, Glu, Pro, Ala, Gly, Trp et Tyr; a vaut 7, b vaut 4 ou 5, c vaut 10 et d vaut 8; il existe des ponts disulfure entre $Cys_1$ et $Cys_3$, $Cys_2$ et $Cys_4$, et $Cys_5$ et $Cys_6$, respectivement; à condition que le polypeptide renferme au moins 10 acides aminés AA différents et qu'aucun acide aminé AA ne soit répété plus de 3 fois consécutivement.

24

EP 0 132 021 B1

**2.** Polypeptide biologiquement actif selon la revendication 1, dans lequel b vaut 4.

**3.** Polypeptides biologiquement actifs ou leurs oligomères de formule:

$$(AA)_n-Cys_1-(AA)_m-Cys_2-(AA)_o-Cys_3-(AA)_p-Cys_4-AA-Cys_5-(AA)_q-Cys_6-(AA)_r$$

dans laquelle AA est un résidu d'acide aminé choisi parmi Val, Ser, His, Phe, Asn, Lys, Asp, Thr, Gln, Arg, Leu, Glu, Pro, Ala, Gly, Trp et Tyr; et n vaut de 4 à 10, m vaut 7, o vaut 4 ou 5, p vaut 10, q vaut 8 et r est un nombre entier valant de 6 à 12; il existe des ponts disulfure entre $Cys_1$ et $Cys_3$, $Cys_2$ et $Cys_4$, et $Cys_5$ et $Cys_6$, respectivement; à condition que le polypeptide renferme au moins 10 acides aminés AA différents et qu'aucun acide aminé AA ne soit répété plus de 3 fois consécutivement.

**4.** Polypeptide biologiquement actif selon la revendication 3, dans lequel o vaut 4 et n et r valent 7.

**5.** Polypeptides biologiquement actifs renfermant au moins une séquence peptidique de formule:
$$-Cys_1-(AA)_a-Cys_2-(AA)_b-Cys_3-(AA)_c-Cys_4-AA-Cys_5-(AA)_d-Cys_6-$$
dans laquelle AA est un résidu d'acide aminé choisi parmi Val, Ser, His, Phe, Ile, Met, Asn, Lys, Asp, Thr, Gln, Arg, Leu, Glu, Pro, Ala, Gly, Trp et Tyr; a vaut 7, b vaut 4, c vaut 10 et d vaut 8; il existe des ponts disulfure entre $Cys_1$ et $Cys_3$, $Cys_2$ et $Cys_4$, et $Cys_5$ et $Cys_6$, respectivement; à condition que le polypeptide renferme au moins 10 acides aminés AA différents et qu'aucun acide aminé AA ne soit répété plus de 3 fois consécutivement.

**6.** Polypeptides biologiquement actifs ou leurs oligomères de formule:

$$(AA)_n-Cys_1-(AA)_m-Cys_2-(AA)_o-Cys_3-(AA)_p-Cys_4-AA-Cys_5-(AA)_q-Cys_6-(AA)_r$$

dans laquelle AA est un résidu d'acide aminé choisi parmi Val, Ser, Ile, Met, His, Phe, Asn, Lys, Asp, Thr, Gln, Arg, Leu, Glu, Pro, Ala, Gly, Trp et Tyr; n vaut de 4 à 10, m vaut 7, o vaut 4, p vaut 10, q vaut 8 et r est un nombre entier valant de 6 à 12; il existe des ponts disulfure entre $Cys_1$ et $Cys_3$, $Cys_2$ et $Cys_4$, et $Cys_5$ et $Cys_6$, respectivement; à condition que le polypeptide renferme au moins 10 acides aminés AA différents et qu'aucun acide aminé AA ne soit répété plus de 3 fois consécutivement.

**7.** Polypeptide, facteur de croissance transformant, ou ses oligomères, de formule:

$$Val-Val-Ser-His-Phe-Asn-R-Cys_1-Pro-Asp-Ser-His-Thr-Gln-R'-Cys_2-Phe-His-Gly-Thr-Cys_3-Arg-R''-Leu-Val-Gln-Glu-Glu-Lys-Pro-Ala-Cys_4-Val-Cys_5-His-Ser-Gly-R'''-Val-Gly-Val-Arg-Cys_6-Glu-His-Ala-Asp-Leu-Leu-Ala$$

dans laquelle R est Asp ou Lys; R' est Phe ou Tyr; R'' est Ser ou Phe; R''' est Phe ou Tyr; et il existe des ponts disulfure entre $Cys_1$ et $Cys_3$, $Cys_2$ et $Cys_4$, et $Cys_5$ et $Cys_6$, respectivement.

**8.** Polypeptide selon la revendication 7, dans lequel R est Asp, R' est Phe, R'' est Ser et R''' est Phe.

**9.** Polypeptide selon la revendication 7, dans lequel R est Lys, R' est Tyr, R'' est Phe et R''' est Tyr.

**10.** Un polypeptide facteur de croissance renfermant un ou plusieurs des fragments peptidiquess suivants:

25

A. Val-Val-Ser-His-Phe-Asn-Lys-Cys-Pro-Asp-Ser-His-Thr-Gln-Tyr-Cys-Phe-His-Gly-Thr-Cys

B. Val-Val-Ser-His-Phe-Asn-Asp-Cys-Pro-Asp-Ser-His-Thr-Gln-Phe-Cys-Phe-His-Gly-Thr-Cys

C. Leu-Val-Gln-Glu-Glu-Lys-Pro-Ala-Cys-Val-Cys-His-Ser-Gly, et

D. Cys-His-Ser-Gly-Tyr-Val-Gly-Val-Arg-Cys-Glu-His-Ala-Asp-Leu-Leu-Ala

11. Oligopeptide antigénique choisi parmi:

A. Val-Val-Ser-His-Phe-Asn-Asp-Cys-Pro-Asp-His-Thr

B. Val-Val-Ser-His-Phe-Asn-Lys-Cys-Pro-Asp-His-Thr

C. Arg-Phe-Leu-Val-Gln-Glu-Glu-Lys-Pro-Ala

D. Arg-Tyr-Leu-Val-Gln-Glu-Glu-Lys-Pro-Ala

E. Cys-His-Ser-Gly-Phe-Val-Gly-Val-Arg-Cys-Glu-His-Ala-Asp-Leu-Leu-Ala

F. Cys-His-Ser-Gly-Tyr-Val-Gly-Val-Arg-Cys-Glu-His-Ala-Asp-Leu-Leu-Ala

12. Oligopeptide, capable de se fixer aux récepteurs cellulaires de facteur de croissance, de formule:
$(AA)_x$-Cys-$(AA)_y$-Gly-R-$(AA)_z$-Arg-Cys-$(AA)_{z'}$,
dans laquelle R est Phe ou Tyr, et AA est un résidu d'acide aminé choisi parmi Val, Asn, His, Ser, Lys, Ile, Gly, Leu, Asp, Asn, Cys, Thr, Ala, Tyr, Pro, Glu, Gln et Arg, et x vaut 0 ou un nombre entier de 1 à 6, y vaut 2, z vaut 3 et z' vaut 0 ou un nombre entier de 1 à 6.

13. Oligopeptide selon la revendication 12, dans lequel x et z' valent 0 et AA est un résidu d'acide aminé choisi parmi Val, His, Ser, Ile, Gly et Asp.

14. Oligopeptide biologiquement actif de formule:
$(AA)_x$-Cys-$(AA)_2$-Gly-$(AA)_2$-Gly-$(AA)_2$-Cys-$(AA)_{z'}$,
dans laquelle AA est un résidu d'acide aminé choisi parmi Phe et les résidus d'acides aminés désignés par AA dans la revendication 12, et les indices x et z' ont les définitions données dans la revendication 12.

15. Oligopeptide selon la revendication 13, choisi dans la classe constituée par:
(1) Cys-His-Ser-Gly-Tyr-Val-Gly-Val-Arg-Cys et
(2) Cys-His-Ser-Gly-Phe-Val-Gly-Val-Arg-Cys

16. Anticorps produits contre les facteurs de croissance polypeptidiques des revendications 7 et 10 ou les oligopeptides antigéniques de la revendication 11.

17. Anticorps selon la revendication 16, marqués avec un marqueur capable de fournir un signal détectable.

18. Anticorps selon la revendication 16, marqués avec un agent cytotoxique.

19. Anticorps produits contre les facteurs de croissance polypeptidiques transformants obtenus à partir des

EP 0 132 021 B1

fluides corporels de mammifères porteurs de tumeurs et producteurs de facteur de croissance transformant, lesdits polypeptides correspondant à l'une quelconque des revendications 1 à 6.

20. Procédé d'isolement d'un facteur de croissance polypeptidique transformant homogène, qui est un polypeptide selon l'une quelconque des revendications 1 à 6, à partir d'un milieu aqueux renfermant, à l'état impur, ledit facteur de croissance polypeptidique transformant, lequel procédé comprend les étapes suivantes:

(1) dialyse du milieu aqueux renfermant, à l'état impur, le facteur de croissance transformant, contre une solution aqueuse d'acide acétique, pour fournir une phase solvant contenant le facteur de croissance polypeptidique transformant, phase que l'on concentre et que l'on clarifie éventuellement;

(2) reconstition de la phase solvant concentrée de l'étape (1) avec une solution aqueuse d'acide acétique et chromatographie par perméation de gel de la solution reconstituée, par passage de ladite solution reconstituée sur une colonne de chromatographie par perméation de gel conditionnée avec une solution aqueuse d'acide acétique et élution avec une solution aqueuse d'acide acétique, pour obtenir des fractions choisies d'éluat renfermant le facteur de croissance polypeptidique transformant dans un état de plus grande pureté, lesdites fractions choisies étant rassemblées et concentrées, pour fournir un produit contenant le facteur de croissance polypeptidique transformant partiellement purifié;

(3) soumission du produit de l'étape (2), contenant le facteur de croissance polypeptidique transformant partiellement purifié, à une chromatographie haute pression séquentielle à polarité de phases inversée, par passage dudit produit, après reconstitution dans une solution aqueuse d'acide trifluoroacétique, à travers une ou plusieurs colonne(s) de silice liée à un hydrocarbure, qui a (ont) été équilibrée(s) avec une solution aqueuse d'acide trifluoroacétique, dans des conditions de chromatographie en phase liquide haute pression, l'élution initiale de la colonne étant réalisée avec un gradient linéaire d'acétonitrile dans une solution aqueuse d'acide trifluoroacétique, et l'élution suivante de la colonne, effectuée sur les fractions rassemblées, contenant le facteur de croissance polypeptidique transformant, de l'étape initiale de chromatographie haute pression, étant réalisée avec un gradient linéaire de 1-propanol dans une solution aqueuse d'acide trifluoroacétique, ledit gradient de 1-propanol augmentant avec des incréments de concentration de 1-propanol assez petits pour fournir le facteur de croissance polypeptidique transformant sous forme d'un pic unique distinct, à l'état de polypeptide homogène.

21. Procédé selon la revendication 20, dans lequel le milieu aqueux renfermant ledit facteur de croissance polypeptidique transformant à l'état impur est un fluide corporel contenant le facteur de croissance transformant ou un milieu aqueux conditionné avec une lignée cellulaire produisant le facteur de croissance transformant.

22. Facteur de croissance polypeptidique transformant homogène obtenu par le procédé selon la revendication 21.

23. Facteur de croissance polypeptidique transformant homogène selon la revendication 22, dans lequel le polypeptide homogène est un facteur de croissance polypeptidique transformant humain.

24. Anticorps produits contre les facteurs de croissance polypeptidiques transformants homogènes de la revendication 22.

25. Anticorps selon l'une des revendications 19 ou 24, marqués avec un marqueur capable de fournir un signal détectable.

26. Anticorps selon l'une des revendications 19 ou 24, marqués avec un agent cytotoxique.

27. Procédé in vitro de détection d'une tumeur maligne chez un hôte humain, comprenant la mise en contact de cellules ou de fluides corporels provenant dudit hôte humain avec un anticorps d'une des revendications 16, 19 ou 24, et la détermination du degré de fixation dudit anticorps auxdites cellules ou aux produits cellulaires présents dans ledit fluide corporel, comme méthode de diagnostic chez un hôte porteur de tumeur maligne.

28. Composition pour le traitement du cancer ou d'une autre maladie proliférative, qui comprend une

27

quantité thérapeutique d'un polypeptide d'une des revendications 7 ou 10 ou un oligopeptide d'une des revendications 12 ou 14, associé à un véhicule pharmaceutiquement acceptable.

29. Composition pour le traitement et/ou la réparation de dommages causés aux cellules ou aux tissus, qui comprend une quantité efficace d'un polypeptide d'une des revendications 1, 3, 5 ou 6, associé à un véhicule pharmaceutiquement acceptable.

30. Peptide biologiquement actif de l'une quelconque des revendications 1, 3, 5 ou 6, pour traiter et/ou réparer des dommages causés aux cellules ou aux tissus.

31. Polypeptide selon la revendication 7, pour traiter et/ou réparer des dommages causés aux cellules ou aux tissus.

32. Anticorps selon l'une quelconque des revendications 16, 19 ou 24, pour détecter une affection maligne chez l'homme.

**Patentansprüche**

1. Biologisch aktive Polypeptide, enthaltend wenigstens eine Peptidsequenz der Formel:

$$-Cys_1-(AA)_a-Cys_2-(AA)_b-Cys_3-(AA)_c-Cys_4-$$
$$AA-Cys_5-(AA)_d-Cys_6-$$

worin jedes AA einen Aminosäurerest, ausgewählt aus Val, Ser, His, Phe, Asn, Lys, Asp, Thr, Gln, Arg, Leu, Glu, Pro, Ala, Gly, Trp und Tyr darstellt; a 7, b 4 oder 5, c 10 und d 8 ist; zwischen $Cys_1$ und $Cys_3$, $Cys_2$ und $Cys_4$ bzw. $Cys_5$ und $Cys_6$ Disulfidbrücken bestehen und mit den Maßgaben, daß die Polypeptide wenigstens zehn verschiedene Aminosäuren AA enthalten und daß keine Aminosäure AA mehr als dreimal hintereinander wiederholt ist.

2. Biologisch aktives Polypeptid nach Anspruch 1, worin b 4 ist.

3. Biologisch aktive Polypeptide oder Oligomere hievon der Formel:

$$(AA)_n-Cys_1-(AA)_m-Cys_2-(AA)_o-Cys_3-(AA)_p-$$
$$Cys_4-AA-Cys_5-(AA)_q-Cys_6-(AA)_r$$

worin AA einen Aminosäurerest, ausgewählt aus Val, Ser, His, Phe, Asn, Lys, Asp, Thr, Gln, Arg, Leu, Glu, Pro, Ala, Gly, Trp und Tyr darstellt; n eine ganze Zahl von 4 bis 10, m 7, o 4 oder 5, p 10, q 8 und r eine ganze Zahl von 6 bis 12 ist; zwischen $Cys_1$ und $Cys_3$, $Cys_2$ und $Cys_4$ bzw. $Cys_5$ und $Cys_6$ Disulfidbrücken bestehen und mit den Maßgaben, daß das Polypeptid wenigstens zehn verschiedene Aminosäuren AA enthält und daß keine Aminosäure AA mehr als dreimal hintereinander wiederholt ist.

4. Biologisch aktives Polypeptid nach Anspruch 3, worin o 4 ist und n und r die Bedeutung 7 haben.

5. Biologisch aktive Polypeptide, enthaltend wenigstens eine Peptidsequenz der Formel:

$$-Cys_1-(AA)_a-Cys_2-(AA)_b-Cys_3-(AA)_c-Cys_4-$$
$$AA-Cys_5(AA)_d-Cys_6-$$

worin AA einen Aminosäurerest, ausgewählt aus Val, Ser, His, Phe, Ile, Met, Asn, Lys, Asp, Thr, Gln, Arg, Leu, Glu, Pro, Ala, Gly, Trp und Tyr darstellt; a 7, b 4, c 10 und d 8 ist; zwischen $Cys_1$ und $Cys_3$, $Cys_2$ und $Cys_4$ bzw. $Cys_5$ und $Cys_6$ Disulfidbrücken bestehen und mit den Maßgaben, daß das Polypeptid wenigstens zehn verschiedene Aminosäuren AA enthält und daß keine Aminosäure AA mehr als dreimal hintereinander wiederholt ist.

6. Biologisch aktive Polypeptide oder Oligomere hievon der Formel:

$$(AA)_n\text{-}Cys_1\text{-}(AA)_m\text{-}Cys_2\text{-}(AA)_o\text{-}Cys_3\text{-}(AA)_p\text{-}Cys_4\text{-}AA\text{-}Cys_5\text{-}(AA)_q\text{-}Cys_6\text{-}(AA)_r \quad ,$$

worin AA einen Aminosäurerest, ausgewählt aus Val, Ser, Ile, Met, His, Phe, Asn, Lys, Asp, Thr, Gln, Arg, Leu, Glu, Pro, Ala, Gly, Trp und Tyr darstellt; n eine ganze Zahl von 4 bis 10, m 7, o 4, p 10, q 8 und r eine ganze Zahl von 6 bis 12 ist; zwischen $Cys_1$ und $Cys_3$, $Cys_2$ und $Cys_4$ bzw. $Cys_5$ und $Cys_6$ Disulfidbrücken bestehen und mit den Maßgaben, daß das Polypeptid wenigstens zehn verschiedene Aminosäuren AA enthält und daß keine Aminosäure AA mehr als dreimal hintereinander wiederholt ist.

7. Wachstumsfaktor transformierendes Polypeptid oder Oligomere hievon der Formel:

$$\text{Val-Val-Ser-His-Phe-Asn-R-}Cys_1\text{-Pro-Asp-Ser-His-Thr-}$$
$$\text{Gln-R'-}Cys_2\text{-Phe-His-Gly-Thr-}Cys_3\text{-Arg-R''-Leu-Val-}$$
$$\text{Gln-Glu-Glu-Lys-Pro-Ala-}Cys_4\text{-Val-}Cys_5\text{-His-Ser-Gly-}$$
$$\text{R'''-Val-Gly-Val-Arg-}Cys_6\text{-Glu-His-Ala-Asp-Leu-Leu-}$$
$$\text{Ala} \quad ,$$

worin R Asp oder Lys; R' Phe oder Tyr; R'' Ser oder Phe; R''' Phe oder Tyr darstellt; und zwischen $Cys_1$ und $Cys_3$, $Cys_2$ und $Cys_4$ bzw. $Cys_5$ und $Cys_6$ Disulfidbrücken bestehen.

8. Polypeptid nach Anspruch 7, worin R Asp, R' Phe, R'' Ser und R''' Phe ist.

9. Polypeptid nach Anspruch 7, worin R Lys, R' Tyr, R'' Phe und R''' Tyr ist.

10. Polypeptidwachstumsfaktor, enthaltend ein oder mehrere der folgenden Peptidfragmente:

A.     Val-Val-Ser-His-Phe-Asn-Lys-Cys-Pro-Asp-Ser-His-Thr-Gln-Tyr-Cys-Phe-His-Gly-Thr-Cys

B.     Val-Val-Ser-His-Phe-Asn-Asp-Cys-Pro-Asp-Ser-His-Thr-Gln-Phe-Cys-Phe-His-Gly-Thr-Cys

C.     Leu-Val-Gln-Glu-Glu-Lys-Pro-Ala-Cys-Val-Cys-His-Ser-Gly  und

D.     Cys-His-Ser-Gly-Tyr-Val-Gly-Val-Arg-Cys-Glu-His-Ala-Asp-Leu-Leu-Ala.

11. Antigenoligopeptid, ausgewählt aus der Klasse bestehend aus:

A.     Val-Val-Ser-His-Phe-Asn-Asp-Cys-Pro-Asp-His-Thr

B.     Val-Val-Ser-His-Phe-Asn-Lys-Cys-Pro-Asp-His-Thr

C.     Arg-Phe-Leu-Val-Gln-Glu-Glu-Lys-Pro-Ala

D.     Arg-Tyr-Leu-Val-Gln-Glu-Glu-Lys-Pro-Ala

E.     Cys-His-Ser-Gly-Phe-Val-Gly-Val-Arg-Cys-Glu-His-Ala-Asp-Leu-Leu-Ala

F.     Cys-His-Ser-Gly-Tyr-Val-Gly-Val-Arg-Cys-Glu-His-Ala-Asp-Leu-Leu-Ala.

12. Oligopeptid mit der Fähigkeit, sich an zelluläre Wachstumsfaktorrezeptoren zu binden und der Formel:
$(AA)_x$-Cys-$(AA)_y$-Gly-R-$(AA)_z$-Arg-Cys-$(AA)_{z'}$,
worin R Phe oder Tyr ist und AA einen Aminosäurerest, ausgewählt aus Val, Asn, His, Ser, Lys, Ile, Gly, Leu, Asp, Asn, Cys, Thr, Ala, Tyr, Pro, Glu, Gln und Arg darstellt, x Null oder eine ganze Zahl von 1 bis 6, y 2, z 3 und z' Null oder eine ganze Zahl von 1 bis 6 ist.

13. Oligopeptid nach Anspruch 12, worin x und z' Null sind und AA einen Aminosäurerest, ausgewählt aus Val, His, Ser, Ile, Gly und Asp darstellt.

14. Biologisch aktives Oligopeptid der Formel:
$(AA)_x$-Cys-$(AA)_2$-Gly-$(AA)_2$-Gly-$(AA)_2$-Cys-$(AA)_{z'}$,
worin AA einen Aminosäurerest, ausgewählt aus Phe und den in Anspruch 12 durch AA bezeichneten Aminosäureresten darstellt und die Indices x und z' wie in Anspruch 12 definiert sind.

EP 0 132 021 B1

**15.** Oligopeptid nach Anspruch 13, ausgewählt aus der Klasse bestehend aus:

(1) Cys-His-Ser-Gly-Tyr-Val-Gly-Val-Arg-Cys und
(2) Cys-His-Ser-Gly-Phe-Val-Gly-Val-Arg-Cys ,

**16.** Antikörper, gebildet auf die Polypeptidwachstumsfaktoren von Anspruch 7 und 10 oder auf die Antigenoligopeptide von Anspruch 11.

**17.** Antikörper nach Anspruch 16, markiert mit einer zur Vorsehung eines erkennbaren Signals fähigen Markierung.

**18.** Antikörper nach Anspruch 16, markiert mit einem cytotoxischen Mittel.

**19.** Antikörper, gebildet auf transformierende Wachstumsfaktorpolypeptide, die aus Körperflüssigkeiten von tumortragenden Säugetieren, die transformierende Wachstumsfaktoren herstellen, wobei die Polypeptide in Übereinstimmung mit einem der Ansprüche 1 bis 6 sind.

**20.** Verfahren zur Isolierung eines homogenen transformierenden Wachstumsfaktorpolypeptids, das ein Polypeptid in Übereinstimmung mit einem der Ansprüche 1 bis 6 ist, aus einem wässerigen Medium, enthaltend das transformierende Wachstumsfaktorpolypeptid in unreiner Form, durch die Verfahrensschritte beinhaltend:
(1) Dialysieren des wässerigen Mediums, das den transformierenden Wachstumsfaktor in unreiner Form enthält, gegen wässerige Essigsäure, wodurch eine Lösungsmittelphase erhalten wird, die das transformierende Wachstumsfaktorpolypeptid enthält, welche Phase konzentriert und gegebenenfalls geklärt wird,
(2) Wiederherstellen der konzentrierten Lösungsmittelphase von Schritt (1) mit wässeriger Essigsäure und Unterziehen der wiederhergestellten Lösung einer Gelpermeationschromatographie durch Aufbringen der wiederhergestellten Lösung auf eine Gelpermeationschromatographiesäule, die mit wässeriger Essigsäure konditioniert ist und Eluieren mit wässeriger Essigsäure, wodurch ausgewählte Fraktionen des transformierenden Wachstumsfaktorpolypeptids in erhöhtem Stadium der Reinheit enthaltender Eluatfraktionen erhalten werden, Vereinigen und Konzentrieren der ausgewählten Fraktionen, wodurch ein teilweise gereinigtes, das transformierende Wachstumsfaktorpolypeptid enthaltende Produkt erhalten wird,
(3) Unterziehen des teilweise gereinigten, transformierenden Wachstumsfaktorpolypeptid enthaltenden Produktes von Schritt (2) einer sequentiellen Umkehrphasen-Hochdruckchromatographie durch Leiten des Produktes nach Wiederherstellung in wässeriger Trifluoressigsäure durch eine oder mehrere kohlenwasserstoffgebundene Kieselerdematrixsäulen, die mit wässeriger Trifluoressigsäure äquilibriert wurden, unter Hochdruckflüssigchromatographiebedingungen, wobei die anfängliche Säuleneluierung unter Verwendung eines linearen Acetonitrilgradienten in wässeriger Trifluoressigsäure durchgeführt wird und die nachfolgende Säuleneluierung an den vereinigten, das transformierende Wachstumsfaktorpolypeptid enthaltenden Fraktionen des anfänglichen Hochdruckchromatographieschrittes unter Verwendung eines linearen 1-Propanolgradienten in wässeriger Trifluoressigsäure durchgeführt wird, wobei der 1-Propanolgradient in genügend kleinen 1-Propanolkonzentrationsinkrementen gesteigert wird, wobei das transformierende Wachstumsfaktorpolypeptid als einzelner, getrennter Peak als homogenes Polypeptid erhalten wird.

**21.** Verfahren gemäß Anspruch 20, worin das wässerige Medium, welches das transformierende Wachstumsfaktorpolypeptid in unreiner Form enthält, eine Körperflüssigkeit, die transformierenden Wachstumsfaktor enthält, oder ein wässeriges Medium ist, das mit einer, den transformierenden Wachstumsfaktor herstellenden Zellinie konditioniert ist.

**22.** Homogenes transformierendes Wachstumsfaktorpolypeptid, erhalten durch das Verfahren nach Anspruch 21.

**23.** Homogenes transformierendes Wachstumsfaktorpolypeptid nach Anspruch 22, worin das homogene Polypeptid ein menschliches transformierendes Wachstumsfaktorpolypeptid ist.

**24.** Antikörper gebildet auf die homogenen transformierenden Wachstumsfaktorpolypeptide von Anspruch

31

22.

25. Antikörper nach Anspruch 19 oder 24, markiert mit einer zur Vorsehung eines erkennbaren Signals fähigen Markierung.

26. Antikörper nach Anspruch 19 oder 24, markiert mit einem cytotoxischen Mittel.

27. In vitro-Methode zur Erkennung von Malignität in einem menschlichen Wirt, beinhaltend das Inberührungbringen von Zellen oder Körperflüssigkeiten des menschlichen Wirts mit einem Antikörper von Anspruch 16, 19 oder 24 und Bestimmung des Bindungsausmaßes des Antikörpers an die Zellen oder zellulären Produkte in der Körperflüssigkeit als charakteristisches Merkmal eines Malignität tragenden Wirts.

28. Zusammensetzung zur Behandlung von Krebs oder anderen proliferativen Erkrankungen, beinhaltend eine therapeutische Menge eines Polypeptids von Anspruch 7 oder 10 oder eines Oligopeptids von Anspruch 12 oder 14, zusammen mit einem pharmazeutisch annehmbaren Träger hiefür.

29. Zusammensetzung zur Behandlung und/oder Ausbesserung von Zell- oder Gewebeschäden, beinhaltend eine wirksame Menge eines Polypeptids von Anspruch 1, 3, 5 oder 6, zusammen mit einem pharmazeutisch annehmbaren Träger hiefür.

30. Biologisch aktives Peptid nach einem der Ansprüche 1, 3, 5 oder 6 zum Behandeln und/oder Ausbessern von Zell- oder Gewebeschäden.

31. Polypeptid nach Anspruch 7 zur Behandlung und/oder Ausbesserung von Zell- oder Gewebeschäden.

32. Antikörper nach einem der Ansprüche 16, 19 oder 24, zur Erkennung von Malignität in einem menschlichen Wirt.